# EUROPEAN PATENT SPECIFICATION

(11) **EP 1 200 406 B1**
(45) Date of publication and mention of the grant of the patent: **24.11.2004**
(21) Application number: 00945917.3
(22) Date of filing: 14.07.2000
(51) Int. Cl.: C07D 211/88, C07D 207/40, C07D 209/54, C07D 221/20, C07D 401/06, A61K 31/4015, A61K 31/4545, A61K 31/403, A61K 31/438, A61P 9/10, A61P 13/10

(54) **CYCLIC AMIDES AND IMIDES HAVING SELECTIVE ANTAGONIST ACTIVITY AT ALPHA-1D ADRENERGIC RECEPTOR**
CYKLISCHE AMIDE UND IMIDE UND DEREN VERWENDUNG ALS ALPHA-1D ADRENERGISCHE REZEPTOR-ANTAGONISTEN
AMIDES ET IMIDES CYCLIQUES POSSEDANT UNE ACTIVITE ANTAGONISTE SELECTIVE AU NIVEAU DU RECEPTEUR ADRENERGIQUE 1D

(30) Priority: 15.07.1999 IT MI991578
(43) Date of publication of application: 02.05.2002
(73) Proprietor: RECORDATI INDUSTRIA CHIMICA E FARMACEUTICA S.p.a., 20148 Milano (IT); RECORDATI S.A., 6830 Chiasso (CH)
(72) Inventor: LEONARDI, Amedeo, I-20154 Milano (IT); BARLOCCO, Daniela, I-20146 Milan (IT); MOTTA, Gianni, I-20030 Barlassina (IT); TESTA, Rodolfo, I-20060 Vignate (IT)
(74) Representative: McKelvey, Ian Edward
(86) International application number: PCT/EP2000/006738
(87) International publication number: WO 2001/005765

(56) References cited:
- EP-A- 0 911 330
- WO-A-00/04012
- MARIA L. LOPEZ-RODRIGUEZ: "1-[omega-(4-Arylpiperazin-1-yl)alkyl]-3-d iphenylmethylene-2,5-pyrrolidinediones as 5HT1A Receptor Ligands: Study of the Steric Requirements of the Terminal Amide Fragment on 5-HT1A Affinity/Selectivity" BIOORGANIC & MEDICINAL CHEMISTRY LETTERS, vol. 8, 1998, pages 581-586, XP002924834

## Description

### BACKGROUND OF THE INVENTION

N,ω-aminoalkyl cyclic imides are reported in the scientific literature and among them the most investigated for its pharmacological activity at the adrenergic receptor is BMY 7378.

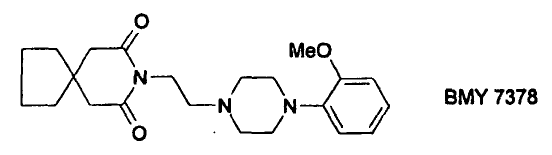

This molecule was first described by Y-H. Wu et al. in *J. Med*. *Chem* 12, 876-881 (1969) and later investigated as a serotonergic-5HT_{1A}-receptor putative antagonist by T. Sharp et al., *Eur. J. Pharmacol*. 176, 331-340 (1990). More recently, A. S. Goetz et al., *Eur. J. Pharmacol.* 272, R5-R6 (1995) reported for BMY 7378 a selective antagonism for the D subtype of the α1-adrenergic receptor.
Further N, ω-aminoalkyl cyclic amides are described in EP 911,330. These have the formula 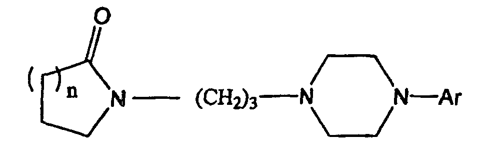 wherein n is 1 or 2 and Ar represents a phenyl ring substituted by halo, alkoxy, alkyl or heteroaryl groups. These compounds are described as therapeutic agents for hypertension, ischemia, cardiovascular and other adrenergic receptors related disorders.

They do not have selectivity amongst the α₁ adrenergic receptors. The pharmacological evidence in the specification is for their use in blood pressure reduction.
WO 00/04012 was published after the priority date of this invention. It describes certain 8-[2-(4-halophenyl-1-piperazinyl)-ethyl]-8-azaspiro[4.5]decane-7,9-diones which are specific for the α_{1D} adrenergic receptor.
The compounds of the invention, described below, essentially include a different substitution pattern at the phenylpiperazine group and also a larger structural variability of the imido and amido rings.
These structural variations give the new compounds the ability to bind and interact selectively with the α_{1D} subtype of the adrenergic receptor, this selectivity also limiting interaction with the 5-HT_{1A} serotoninergic receptor, differently from the previously-known α_{1D}-adrenoceptor-"selective" antagonist named BMY 7378, which is endowed with very high affinity for this serotonergic receptor and hence is devoid of selectivity for α_{1D} vs. 5-HT_{1A} receptors as shown below in Table 1 in Example 31.

### SUMMARY OF THE INVENTION

In one aspect the invention is directed to compounds of formula I. 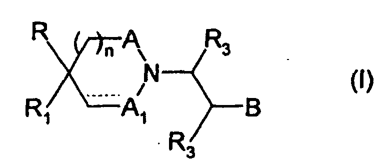 wherein
each of R and R₁ independently represents a hydrogen atom or a lower alkyl group having from 1 to 4 carbon atoms, or R and R₁ together form an alkylene bridge ((CH₂)₂₋₆);
n is an integer from 0 to 1;
...... represents a single or a double bond;
A represents a carbonyl group or a methylene group;
A₁ represents a carbonyl group or a methylene group or a methyne group;
each R₃ independently represents a hydrogen atom or an alkyl group having from 1 to 4 carbon atoms;
B represents one of the following groups: 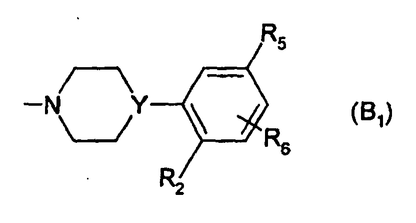 wherein:
Y represents a nitrogen atom or a methyne group;
R₂ represents a halogen atom or a lower alkyl group (C₁-C₄) or a cyano group;
R₅ represents a halogen atom or a lower alkyl group (C₁-C₄) or a polyfluoroalkyl group or a nitro group;
R₆ represents a hydrogen or halogen atom; 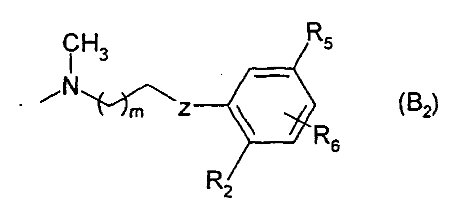
wherein
m is an integer from 1 to 3;
Z represents an oxygen or sulphur atom or an amino or methylamino group;
R₂, R₅ and R₆ have the above meanings;
with the proviso that if R and R₁ together form an alkylene bridge, A and A₁ both represent carbonyl groups, n is 1, the heterocyclic ring is saturated and Y represents a nitrogen atom, then not more than one of R₂, R₅ and R₆ represents a fluorine atom and R₂ and R₅ do not simultaneously represent a methyl group and a chlorine atom respectively.

Preferably R₃ represents a hydrogen atom or a methyl group.

Preferably R₂ represents a fluorine, chlorine, bromine or iodine atom or a methyl or cyano group.

Preferably R₅ represents a fluorine, chlorine, bromine or iodine atom or a trifluoromethyl or nitro group.

Preferably R₆ represents a hydrogen atom or a fluorine atom at position 4 of the phenyl ring.

Preferably R and R₁ together represent a trimethylene, tetramethylene, pentamethylene or hexamethylene group.

Preferably A and A₁ both represent carbonyl groups, or one of A and A₁ represents a carbonyl group and the other of A and A₁ is a methylene unit.

Preferably B represents a B₁ group wherein Y is a nitrogen atom, R₂ and R₅ both represent chlorine atoms.

The invention further provides pharmaceutical compositions comprising a compound of formula I, an enantiomer, diastereoisomer, N-oxide or pharmaceutically acceptable salt of such a compound in admixture with a pharmaceutically acceptable diluent or carrier.

### DETAILED DESCRIPTION OF THE INVENTION

All patents, patent applications, and literature references cited in this application are incorporated by reference in their entirety.

The adrenergic antagonistic activity of the compounds of the invention renders them useful as agents acting on body tissues particularly rich in α_{1D}-adrenergic receptors (such as urinary bladder, blood vessels, etc.). Accordingly, selective antiadrenergic compounds within the invention established as such on the basis of their receptor binding profile, can be useful therapeutic agents for the treatment, for example, of micturition problems associated with unstable bladder and cardiovascular problems due to skeletal-muscle arteriole constriction, or to inhibit smooth-muscle cell proliferation, such as in atherosclerosis.

Pharmacological, biochemical and radioligand binding studies evidenced three different α₁-receptors subtypes with a high affinity for prazosin, namely α_{1A}- (α₁ₐ-), α_{1B}- (α_{1b}-) and α_{1D}- (α_{1d}-), with lower case subscripts being used for recombinant receptors and upper case subscripts for receptors in native tissues (Hieble et al., Pharmacol. Rev. 47: 267-270, (1995)).

The functional involvement of α_{1D}-adrenoceptor in detrusor instabilty secondary to bladder outlet obstruction has been recently reported (T. Broten et al., FASEB Journal 12 (4). A445 (1998)). The relevance of the α_{1D} adrenergic subtype in mediating constriction of rat skeletal muscle arterioles was reported by C. J. Leech et al. (Am. J. Physiol. 270, 4710-722 (1996) and mediation by the α_{1D} adrenoceptor of protein synthesis in arterial smooth muscle leading to abnormal smooth-muscle cell growth was also reported by Xin et al., Mol. Pharmacol. 51, 764-775 (1997).

The selectivity of the compounds of the invention for the α_{1D} receptor should make them effective in the above cited diseases, such as urinary incontinence, vasoconstriction and atherosclerosis, without inducing significant undesired effects on blood pressure, due also to their lower affinity for the 5-HT_{1A} serotonergic receptors. Indeed urapidil (6-{[3-[4-(2-methoxyphenyl)-1-piperazinyl]-propyl]-amino}-1,3-dimethyl-2,4(1*H*,3*H*)-pyrimidinedione), an α₁-antagonist with no selectivity for the α₁ subtypes and good affinity for the 5-HT_{1A} receptor, is known to reduce blood pressure by sympathoinhibitory action caused by activation of 5-HT_{1A} receptors (A. G. Ramage, *Brit. J. Pharmacol*. 102, 998-1002 (1991)). Hence, compounds of the invention could be used without causing undesired hypotensive effects and without interfering with the effect of antihypertensive drugs, when this therapy is needed.

Screening candidate compounds to identify compounds useful in practising the present invention involves measuring the specific binding activity of the compounds towards the different α₁-adrenergic-receptor subtypes, namely the α₁ₐ, α_{1b} and α_{1d} subtypes, according to the method of Testa et al., *Pharmacol. Comm.* 6, 79-86 (1995). By this method a measure of affinity to the human recombinant subtypes of the α₁ adrenoceptor by radioreceptor binding can be obtained.

A detailed description is given in Example 32, where measurement of affinity for the 5-HT_{1A} receptor evaluated by the same approach (radioreceptor binding on recombinant human receptor) is also described.

In addition to affinity by radioreceptor binding also functional antagonism at the α₁ adrenoceptor subtypes can be evaluated, in order to confirm functional selectivity of the compounds of the invention. To this purpose, inhibition of contraction induced by an α₁ adrenoceptor agonist in selected organs or tissues can be assessed. In particular by using noradrenaline and the following tissues, affinity for the α₁ adrenoceptor subtypes can be evaluated:
■ rat aorta, α_{1D} subtype (Testa R. et al., Life Sci. 57, PL159, 1995).
■ epididymal rat vas deferens, α_{1A} subtype (Burt R. P. et al., Br. J. Pharmacol. 115, 467 (1995)).
■ guinea pig spleen, α_{1B} subtype (Eltze M., Eur. J. Pharmacol. 260, 211 (1994)).
■ rabbit aorta preincubated with CEC, α_{1L} subtype (Oshita M. et al., Br J. Pharmacol. 108, 1071 (1993)).

A detailed description of the functional affinity evaluation for the compounds of the invention is given in Example 33.

Once a compound is identified as possessing the desired selectivity characteristics, namely an affinity for the α_{1d} subtype at least 10-fold higher than for the other studied receptors, its pharmacological activity can be confirmed using one or more animal model systems for studying urinary incontinence. A useful animal model system includes without limitation, cystometry in bladder-outlet obstructed rats. The details of this model are given in Example 34.

The compounds of the invention may be prepared by a number of methods known in the art from known starting materials or starting materials that may be prepared by conventional methods.

One method of preparing the cyclic imides of the invention comprises alkylating an amine BH, in which B may be B₁ or B₂, with a haloalkyl intermediate of formula 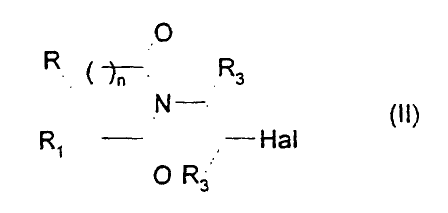 where R, R₁, R₃ and n have the meanings given above and Hal can be, for example, a bromine or chlorine atom. The reaction may be carried out without solvent, in the presence of a base, such as triethylamine (TEA) or diisopropylethylamine (DIPEA), at 100-180°C.

The starting intermediates II, in which R₃ is hydrogen, may be prepared by methods known in the literature, starting from the corresponding imides and 1,2-dihaloethanes. Intermediates II, in which one of R₃ is a lower alkyl group may be prepared by a process such as that exemplified below: 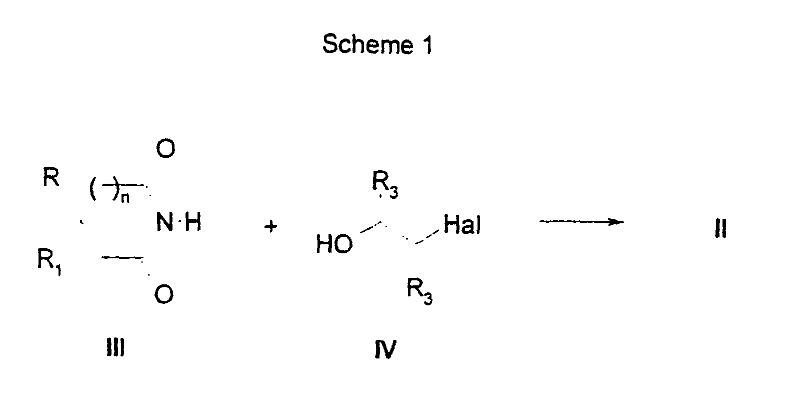 where R, R₁ and n are as defined above, and Hal represents a halogen atom, particularly a chlorine or bromine atom.

The intermediate imides III may be reacted with haloalcohols IV under Mitsunobu conditions using triphenylphosphine and diethyl azodicarboxylate in a polar aprotic solvent, such as dimethylformamide (DMF) or tetrahydrofuran (THF), at 10-80°C.

Intermediates BH in which B is B₁ as follows: 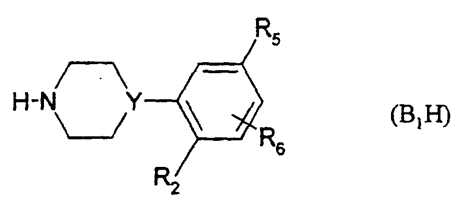 wherein Y is nitrogen and R₂, R₅ and R₆ have the meanings as defined above, are commercially available or may be prepared starting from the corresponding anilines 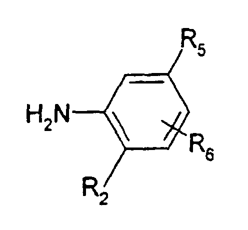 by reaction with *bis*-2-chloroethylamine in the presence of a base, such as sodium or potassium carbonate, and of a promoter, such as potassium iodide, in a polar protic solvent, such as *n*-butyl alcohol or *i*-amyl alcohol. at reflux temperature. Non-commercially-available anilines may be prepared by reduction of the corresponding nitro compounds by methods well known in the literature, for example by using tin (II) chloride in refluxing alcohols.

Intermediates B₁H as above may also be prepared starting from the corresponding piperazines in which R₅ and R₆ are hydrogen. For example B₁H in which R₅ is iodine may be prepared by iodination of the appropriate arylpiperazine using iodine chloride in a polar protic solvent, such as acetic acid, at 10-40°C.

Another method of preparing the compounds of the invention comprises acylating an amine BCH₂CH₂NH₂, in which B is B₁ or B₂, with an anhydride of formula 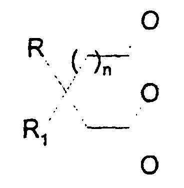 where R, R₁ and n have the meanings given above.

The reaction may be carried out in an apolar aprotic solvent, such as toluene, in the presence of a promoting agent, such as *p*-toluenesulphonic acid, at 80°C to reflux.

Intermediates BCH₂CH₂NH₂, in which B is B₁ of the formula: 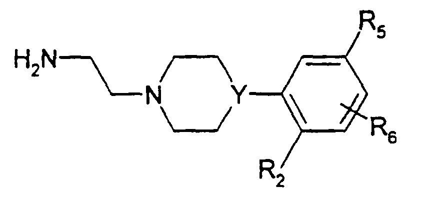 in which Y is nitrogen and R₂, R₅ and R₆ have the meanings defined above, may be prepared according to the following scheme 2.

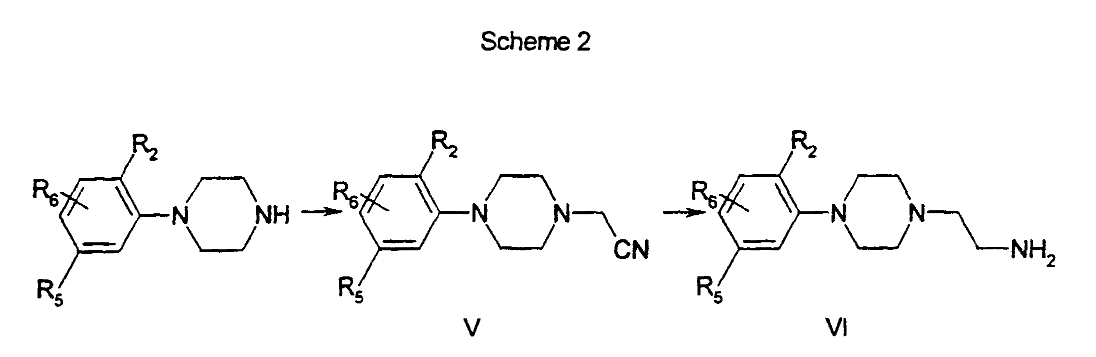

The appropriate arylpiperazines may be alkylated with chloroacetonitrile to give intermediates V. The reaction may be carried out in an aprotic apolar solvent, such as toluene, or in a polar solvent, such as DMF, un the presence of a base, such as potassium carbonate or DIPEA, at 60-120°C. Intermediates V can then be reduced to amines VI. The reaction may be carried out using a reducing agent, such as LiAlH₄ or BH₃-Me₂S, in a polar aprotic solvent, such as diethyl ether or THF, at -10/20°C.

Intermediates BCH₂CH₂NH₂, in which B is B₁, of the formula: 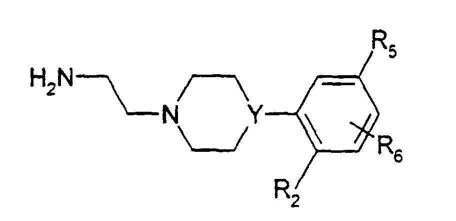 where Y is CH and R₂, R₅ and R₆ have the meanings defined above, may be prepared according to the following Scheme 3.

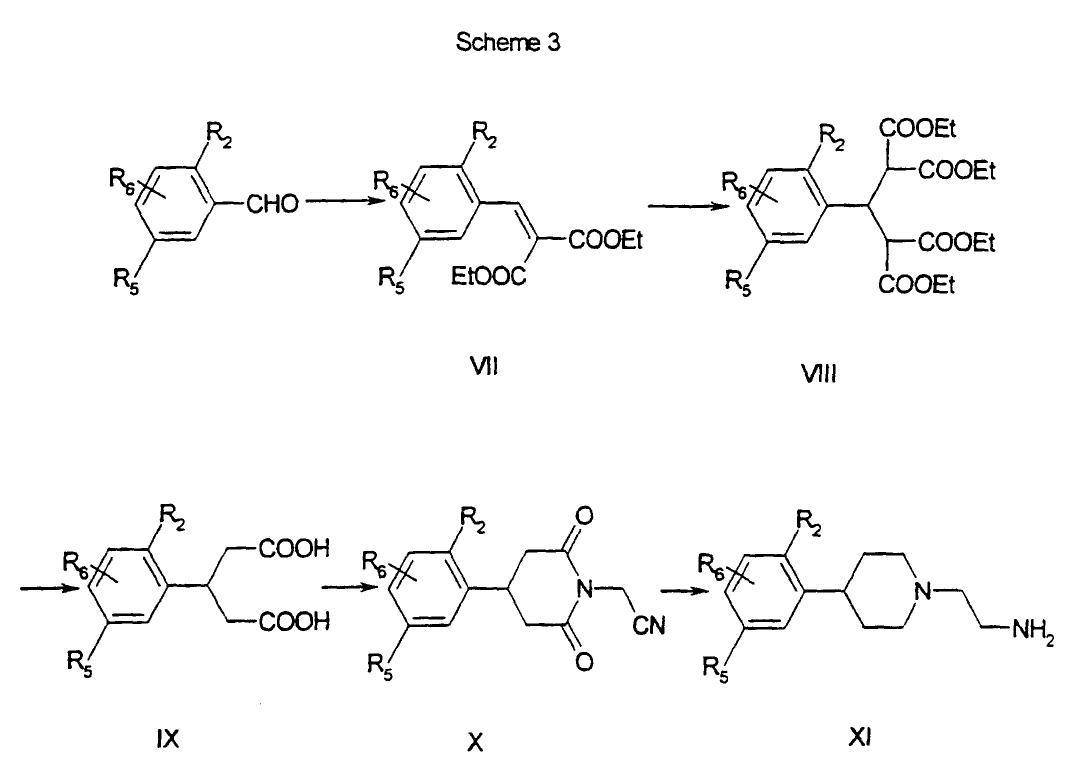

The appropriate aldehydes may be reacted with diethyl malonate in the presence of a Lewis acid, such as AlCl₃, without solvent, at 0-30°C, to give intermediates VII, which are further reacted with diethyl malonate at a higher temperature (at 40-100°C) to give intermediates VIII. The following decarbethoxylation can be carried out in an acidic medium, such as HBr, in water at 80°C to reflux. The diacid intermediate IX may be condensed with aminoacetonitrile in the presence of a condensing agent, such as dicyclohexylcarbodiimide (DCC), in the presence of a base, such as TEA, in a polar aprotic solvent, such as DMF, at 10-40°C, with treatment of the crude in dehydrating conditions, such as acetic anhydride, at 80-110°C, to give intermediates X. These intermediates may be reduced using a metal hydride, such as LiAlH₄ or BH₃-Me₂S, in a polar aprotic solvent, such as diethyl ether or THF, at 20°C to reflux to give intermediates XI.

Intermediates BH, in which B is B₂ of the formula: 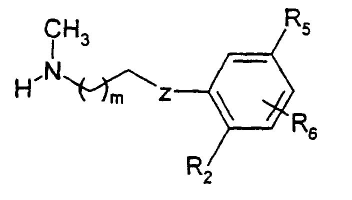 in which m, R₂, R₅ and R₆ have the meanings defined above and Z is NH or NCH₃, may be prepared according to the following scheme 4.

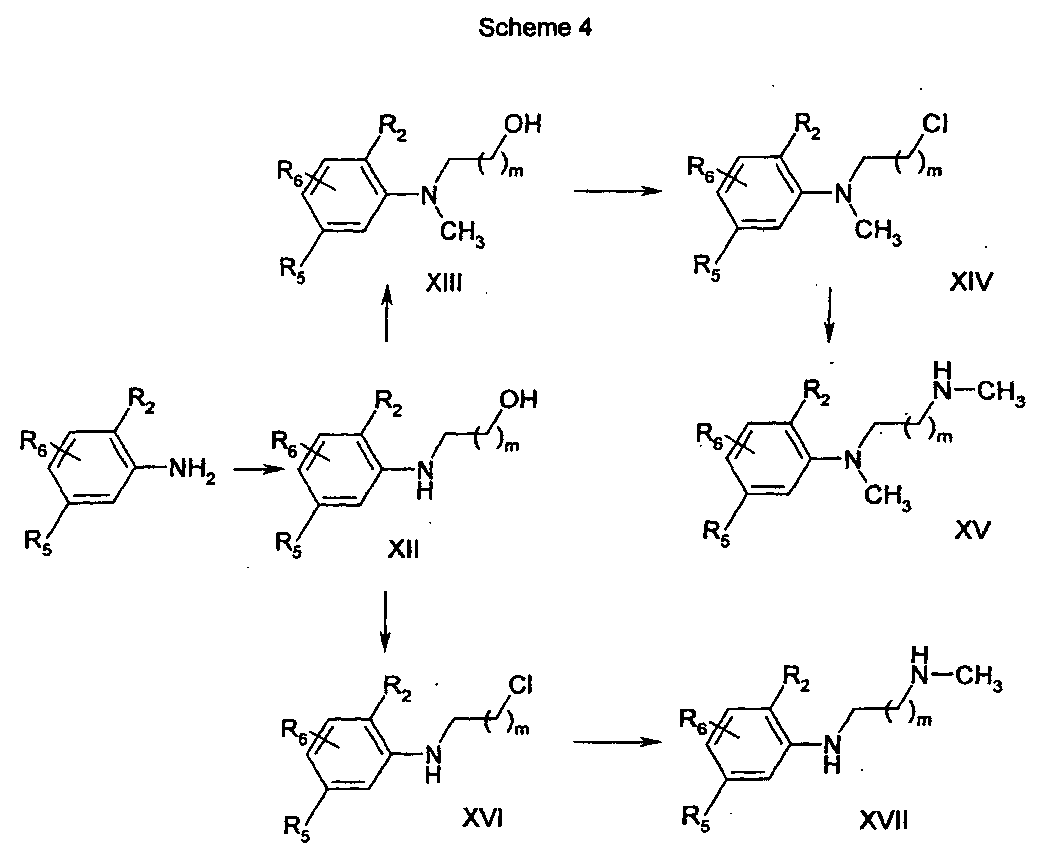

The appropriate anilines may be alkylated by an ω-haloalkyl alcohol (Hal-CH₂(CH₂)ₘ-OH) in the presence of a proton acceptor, such as TEA, at 100-160°C without solvent to give intermediates XII. Intermediates XII are then N-methylated to give the tertiary amines XIII. This reaction may be carried out using formaldehyde in the presence of a reducing agent, such as formic acid, in a polar solvent, such as water, at 100-120°C.

The intermediate aminoalcohols XII and XIII are independently chlorinated to give intermediates XVI and XIV. The reaction may be carried out using a chlorinating agent, such as SOCl₂, in a chlorinated solvent, such as chloroform or dichloromethane, in the presence of a promoter, such as DMF, at 20°C to reflux. Intermediates XIV and XVI are then used to alkylate methylamine to give intermediates XV and XVII respectively. The reaction may be carried out in an autoclave at 60-100°C without solvent.

Intermediates BH, in which B is B₂, as follows: 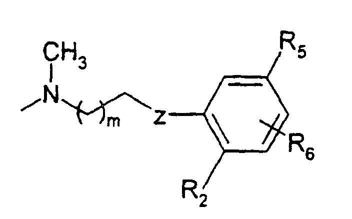 where m, R₂, R₅ and R₆ have the meanings defined above and Z is oxygen or sulphur, may be prepared according to the following scheme 5.

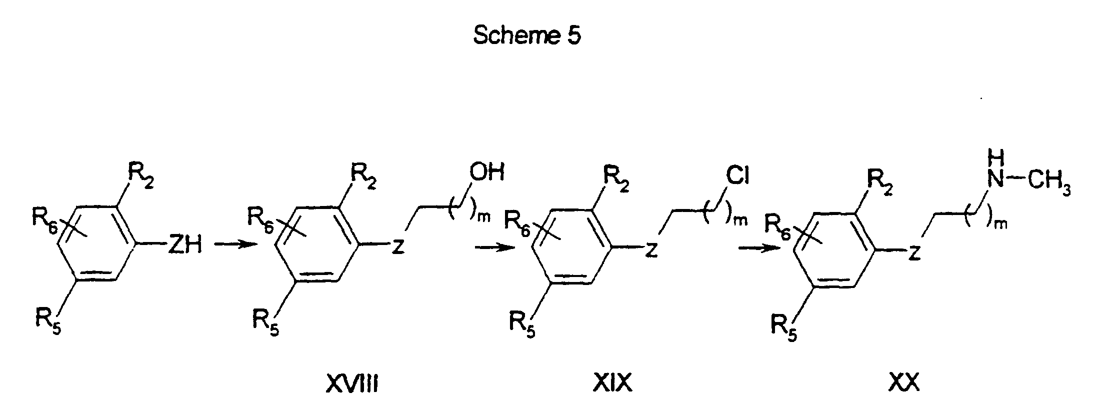

The appropriate phenols or thiophenols may be alkylated by Hal-CH₂(CH₂)ₘ-OH in an apolar aprotic solvent such as toluene, or in a polar one such as DMF, optionally in the presence of a proton acceptor, such as TEA or potassium carbonate, at 80-120°C to give intermediates XVIII. These are chlorinated to give XIX and used to alkylate methylamine to give XX using the conditions described for analogues in scheme 4.

Type B₂ intermediates of formula XV, XVII or XX may be converted into the corresponding derivatives of formula B₂-CH₂CH₂NH₂ using the same synthetic methods shown in scheme 2 for the B₁H piperazine derivatives.

Another method of preparing the compounds of the invention is exemplified in the following scheme 6. 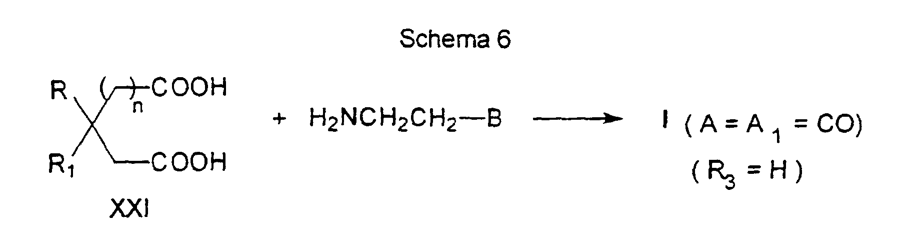 where R, R₁, n and B have the meanings defined above.

The reaction may be carried out first by amidification in the presence of a condensing agent, such as DCC, in a polar aprotic solvent, such as DMF, at 10-40°C, followed by dehydratation using an anhydride, such as acetic anhydride, without solvent at 80-120°C.

Another method of preparing the compounds of the invention is depicted in the following scheme 7. 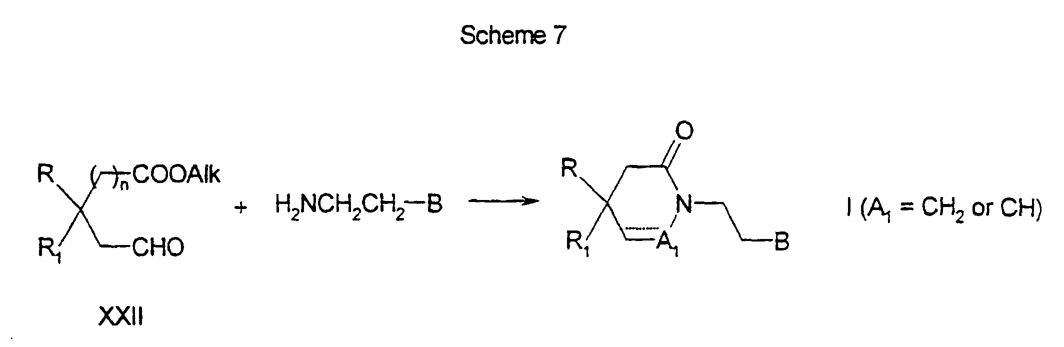 where R, R₁ and B have the meanings defined above, Alk is a lower alkyl group (C₁₋₂) and n is 1.

To obtain compounds I in which ...... is a single bond and A₁ is a methylene group, the reaction may be carried out under reductive conditions using a metal borohydride, such as NaBH₄, in a polar protic solvent, such as methanol, at -5/25°C.

To obtain compounds in which ...... is a double bond and A₁ is a methyne group the reaction may be carried out in an apolar aprotic solvent, such as toluene, in the presence of an acid catalyst, such as *p*-toluenesulphonic acid, at reflux temperature distilling the azeotrope.

Intermediates XXII as defined above may be prepared according to the following scheme 8.

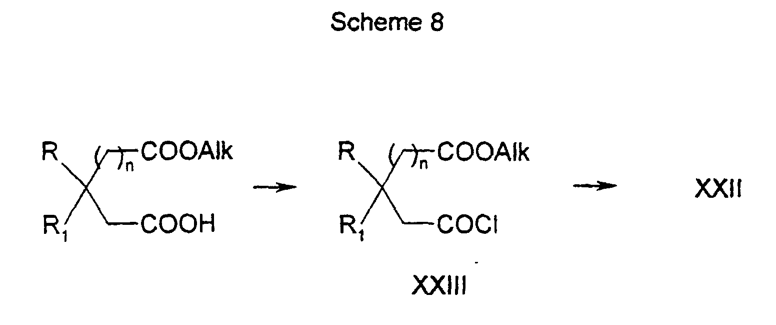

The appropriate diacid monoesters are chlorinated to give intermediates XXIII. The reaction may be carried out using a chlorinating agent, such as SOCl₂ or oxalyl chloride, in a polar aprotic solvent, such as dichloromethane or toluene, in the presence of catalytic amounts of a promoting agent, such as DMF, at 5-25°C. These intermediates are then reduced to give XXII. This reaction may be carried out using a reducing agent, such as hydrogen, in a polar aprotic solvent, such as acetone, in the presence of a catalyst, such as palladium on charcoal, and of a base, such as TEA or DIPEA, at 10-25°C.

In a further method for the preparation of the compounds of the invention, a diester of the general formula 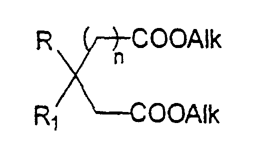 where R, R₁ and n are as above defined is condensed with an amine BCH₂CH₂NH₂ in which B is as above defined. The condensation may be effected without solvent at a temperature ranging from 80 to 160°C.

A method of preparing the compounds of the invention in which A and A₁ are methylene groups and ...... is a single bond consists of a reduction of compounds I in which A and A₁ are carbonyl groups. The reaction may be carried out using a metal borohydride, such as NaBH₄, in the presence of a Lewis catalyst, such as boron trifluoride etherate, in a polar aprotic solvent, such as THF, at -5/25°C.

### SYNTHESIS OF THE COMPOUNDS OF THE INVENTION

### Example 1

### 8-{2-[4-(2,5-Dichlorophenyl)-1-piperazinyl]-ethyl}-8-azaspiro[4.5]decane-7,9-dione

A mixture of 0.27 g (1 mmol) of 8-(2-bromoethyl)-8-azaspiro[4.5]decane-7,9-dione prepared as described by Podona et al., *J. Med. Chem*. 37, 1779-1793 (1994), 0.34 g (1.5 mmol) of 1-(2,5-dichlorophenyl)-piperazine, prepared according to Ratouis R. et al. *J. Med. Chem*. 8, 104-107 (1965), and 0.15 g (1.5 mmol) of triethylamine (TEA) was heated at 180°C for 30 minutes. After cooling to room temperature, the crude was purified by flash chromatography eluting with toluene:acetone 9:1 to give 0.22 g (51%) of the title compound. M.p. 149-150°C.
¹H-NMR (CDCl₃, δ): 1.43-1.59 (m, 4H, CH₂s of spiro ring), 1.61-1.78 (m, 4H, CH₂s of spiro ring), 2.60 (s, 4H, 2 CH₂CO), 2.78-2.98 (m, 10H, CONCH₂CH₂N and piperazine CHs), 4.10 (t, 2H, CONCH₂CH₂N), 6.87-6.97 (m, 2H, phenyl H4 and H6), 7.18-7.24 (m, 1H, phenyl H3).
The following salts were also prepared:
monomethanesulphonate hemihydrate, m.p. 189-190°C (CH₃CN).
dimethanesulphonate, m.p. 189-191°C (CH₃CN);

### Example 2

### 1-{2-[4-(2,5-Dichlorophenyl)-1-piperazinyl]-ethyl}-piperidinyl-2,6-dione.

The title compound was prepared according to the method used in Example 1, but using 1-(2-bromoethyl)-piperidinyl-2,6-dione, prepared according to Podona T. et al., *Tetrahedron* 49, 4619-4626 (1993), instead of 8-(2-bromoethyl)-8-azaspiro[4.5]decane-7,9-dione. Yield 25%.
¹H-NMR (CDCl₃, δ): 1.83-2.02 (m, 2H, CH₂CH₂CO), 2.56 (t, 2H, CONCH₂CH₂N), 2.59-2.78 (m, 8H, , 2 CH₂CO and piperazine CHs), 2.94-3.19 (m, 4H, piperazine CHs), 3.96 (t, 2H, CONCH₂CH₂N), 6.85-6.99 (m, 2H, phenyl H4 and H6), 7.17-7.26 (m, 1H, phenyl H3).

### Example 3

### 8-{2-[4-(2-Chloro-5-methylphenyl)-1-pirerazinyl]-ethyl}-8-azaspiro[4.5]decane-7,9-dione.

The title compound was prepared according to the method described in Example 1, but using 1-(2-chloro-5-methylphenyl)-piperazine, prepared according to Thunus L. et al., *Ann. Pharm. Fr.* 38, 353-358 (1980), instead of 1-(2,5-dichlorophenyl)-piperazine. The mixture was heated at 160°C for 30 minutes and purification was carried out by elution with toluene:acetone 97:3. Yield 73%, m.p. 139-140°C.
¹H-NMR (CDCl₃, δ): 1.47-1.59 (m, 4H, CH₂s of spiro ring), 1.71-1.80 (m, 4H, CH₂s of spiro ring), 2.31 (s, 3H, CH₃), 2.58 (t, 2H, CONCH₂CH₂N), 2.62 (s, 4H, 2 CH₂CO), 2.64-2.78 (m, 4H, piperazine CHs), 2.97-3.09 (m, 4H, piperazine CHs), 3.98 (t, 2H, CONCH₂CH₂N), 6.68-6.83 (m, 2H, phenyl CHs), 7.17-7.22 (m, 1H, phenyl CH).

### Example 4

### 8-{2-[4-(2-Fluoro-5-methylphenyl)-1-piperazinyl]-ethyl}-8-azaspiro[4.5]decane-7.9-dione.

### 1-(2-Fluoro-5-methylphenyl)-piperazine (4A)

A mixture of 0.16 g (1 mmol) of 2-fluoro-5-methylaniline, 0.18 g (1 mmol) of *bis*-(2-chloroethyl)-amine hydrochloride, 0.14 g (1 mmol) of potassium carbonate, 0.08 g (0.5 mmol) of potassium iodide and 5 ml of *n*-butyl alcohol was stirred at reflux for 72 hours. After cooling to room temperature, the solvent was evaporated and the residue treated with water (20 ml) and extracted with dichloromethane. The purification was carried out by flash chromatography eluting with dichloromethane:2N-methanolic-ammonia 96:4 to give 0.17 g of the title compound. Yield 86%.
¹H-NMR (CDCl₃, δ): 2.17 (s, 1H, NH), 2.31 (s, 3H, CH₃), 3.21-3.37 (m, 8H, piperazine CHs), 6.69-6.82 (m, 2H, phenyl CHs), 6.84-6.97 (m, 1H, phenyl CH).

### 8-{2-[4-(2-Fluoro-5-methylphenyl)-1-piperazinyl]-ethyl}-8-azaspiro[4.5]decane-7,9-dione

The title compound was prepared according to the method used in Example 3, but using the above intermediate 4A instead of 1-(2-chloro-5-methylphenyl)-piperazine. Purification was carried out by elution with toluene:acetone 93:7. Yield 59%, m.p. 103-104°C.
¹H-NMR (CDCl₃, δ): 1.24-1.58 (m, 4H, CH₂s of spiro ring), 1.59-1.78 (m, 4H, CH₂s of spiro ring), 2.28 (s, 3H, CH₃), 2.51 (t, 2H, CONCH₂CH₂N), 2.58 (s, 4H, 2 CH₂CO), 2.60-2.77 (m, 4H, piperazine CHs), 2.92-3.09 (m, 4H, piperazine CHs), 3.97 (t, 2H, CONCH₂CH₂N), 6.62-6.77 (m, 2H, phenyl CHs), 6.78-6.90 (m, 1H, phenyl CH).

### Example 5

### 8-{2-[4-(2,5-Dimethylphenyl)-1-piperazinyl]-ethyl}-8-azaspiro[4.5]decane-7,9-dione

The title compound was prepared according to the method used in Example 3, but using 1-(2,5-dimethylphenyl)-piperazine, instead of 1-(2-chloro-5-methylphenyl)-piperazine. Purification was carried out by elution with toluene:acetone 9:1. Yield 76%, m.p. 128-129°C.
¹H-NMR (CDCl₃, δ): 1.49-1.62 (m, 4H, CH₂s of spiro ring), 1.68-1.80 (m, 4H, CH₂s of spiro ring), 2.24 (s, 3H, CH₃), 2.33 (s, 3H, CH₃), 2.58 (t, 2H, CONCH₂CH₂N), 2.60-2.77 (m, 8H, 2 CH₂CO and piperazine CHs), 2.81-2.87 (m, 4H, piperazine CHs), 3.38 (t, 2H, CONCH₂CH₂N), 6.74-6.83 (m, 2H, phenyl CHs), 7.00-7.09 (m, 1H, phenyl CH).

### Example 6

### 8-{2-[4-(2-Fluoro-5-trifluoromethylphenyl)-1-piperazinyl]-ethyl}-8-azaspiro[4.5]decane-7,9-dione

### 1-(2-Fluoro-5-trifluoromethyl)-piperazine (6A)

This compound was prepared according to the method described in Example 4 for intermediate 4A, but using 2-fluoro-5-trifluoromethylaniline instead of 2-fluoro-5-methylaniline. Yield 9%.
¹H-NMR (CDCl₃, δ): 2.08 (s, 1H, NH), 2.89-3.18 (m, 8H, piperazine CHs), 6.95-7.22 (m, 3H, phenyl CHs).

### 8-{2-[4-(2-Fluoro-5-trifluoromethylphenyl)-1-piperazinyl]-ethyl}-8-azaspiro[4.5]decane-7,9-dione

The title compound was prepared according to the method described in Example 3, but using the above intermediate 6A instead of 1-(2-chloro-5-methylphenyl)-piperazine. Purification was carried out by elution with toluene:acetone 9:1. Yield 81%, m.p. 85-86°C.
¹H-NMR (CDCl₃, δ): 1.47-1.60 (m, 4H, CH₂s of spiro ring), 1.68-1.79 (m, 4H, CH₂s of spiro ring), 2.57 (t, 2H, CONCH₂CH₂N), 2.62 (s, 4H, 2 CH₂CO), 2.64-2.78 (m, 4H, piperazine CHs), 3.04-3.17 (m, 4H, piperazine CHs), 3.95 (t, 2H, CONCH₂CH₂), 7.00-7.22 (m, 3H, phenyl CHs).

### Example 7

### 8-{2-[4-(2,5-Dibromophenyl)-1-piperazinyl]-ethyl}-8-azaspiro[4.5]decane-7,9-dione

### 1-(2,5-Dibromophenyl)-piperazine (7A)

This compound was prepared according to the method described in Example 4 for intermediate 4A, but using 2,5-dibromoaniline instead of 2-fluoro-5-methylaniline. Yield 10%.
¹H-NMR (CDCl₃, δ): 1.89 (s, 1H, NH), 2.92-3.16 (m, 8H, piperazine CHs), 7.02 (dd, 1H, phenyl H4), 7.16 (d, 1H, phenyl H6), 7.22 (d, 1H, phenyl H3).

### 8-{2-[4-(2,5-Dibromophenyl)-1-piperazinyl]-ethyl}-8-azaspiro[4.5]decane-7,9-dione

The title compound was prepared according to the method used in Example 3, but using the above intermediate 7A instead of 1-(2-chloro-5-methylphenyl)-piperazine. Purification was carried out eluting with toluene:acetone 92:8. Yield 55%, m.p. 159-160°C.
¹H-NMR (CDCl₃, δ): 1.43-1.58 (m, 4H, CH₂s of spiro ring), 1.68-1.80 (m, 4H, CH₂s of spiro ring), 2.56 (t, 2H, CONCH₂CH₂N), 2.61 (s, 4H, 2 CH₂CO), 2.63-2.77 (m, 4H, piperazine CHs), 2.93-3.08 (m, 4H, piperazine CHs), 3.98 (t, 2H, CONCH₂CH₂N), 7.01 (dd, 1H, phenyl H4), 7.12 (d, 1H, phenyl H6), 7.39 (d, 1H, phenyl H3).

### Example 8

### 8-{2-[4-(5-Chloro-2-cyanophenyl)-1-piperazinyl]-ethyl}-8-azaspiro[4.5]decane-7,9-dione

### 1-(5-Chloro-2-cyanophenyl)-piperazine (8A)

This compound was prepared according to the method described in Example 4 for intermediate 4A, but using 2-amino-4-chlorobenzonitrile instead of 2-fluoro-5-methylaniline. Yield 9%.
¹H-NMR (CDCl₃, δ): 2.77 (s, 1H, NH), 2.91-3.10 (m, 8H, piperazine CHs), 6.58-6.86 (m, 2H, phenyl CHs), 7.19-7.31 (m, 1H, phenyl CH).

### 8-{2-[4-(5-Chloro-2-cyanophenyl)-1-piperazinyl]-ethyl}-8-azaspiro[4.5]decane-7,9-dione

The title compound was prepared according to the method used in Example 1, but using the above intermediate 9A instead of 1-(2,5-dichlorophenyl)-piperazine. Yield 23%.
¹H-NMR (CDCl₃, δ): 1.43-1.60 (m, 4H, CH₂s of spiro ring), 1.63-1.80 (m, 4H, CH₂s of spiro ring), 2.55 (t, 2H, CONCH₂CH₂N), 2.61 (s, 4H, 2 CH₂CO), 2.63-2.77 (m, 4H, piperazine CHs), 3.11-3.23 (m, 4H, piperazine CHs), 3.96 (t, 2H, CONCH₂CH₂N), 6.94 (dd, 2H, phenyl CHs), 7.44 (d, 1H, phenyl CH).

### Example 9

### 8-{2-[4-(2-Chloro-5-fluorophenyl)-1-piperazinyl]-ethyl}-8-azaspiro[4.5]decane-7,9-dione

### 2-Chloro-5-fluoroaniline (9A)

A mixture of 0.17 g (1 mmol) of 2-chloro-5-fluoronitrobenzene, 1.13 g (5 mmol) of tin (II) chloride dihydrate and 10 ml of EtOH was stirred at 70°C for 0.5 hours. After cooling to room temperature, the mixture was poured into H₂O (30 ml), alkalinized with 10% Na₂CO₃ and extracted with CH₂Cl₂. The organic layer was dried (Na₂SO₄) and evaporated to dryness to give 0.12 g (84.5%) of the desired compound.
¹H-NMR (CDCl₃, δ): 4.16 (s, 2H, NH₂), 6.51-6.77 (m, 2H, phenyl CHs), 7.09-7.21 (m, 1H, phenyl CH).

### 1-(2-Chloro-5-fluorophenyl)-piperazine (9B)

This compound was prepared according to the method described in Example 4 for intermediate 4A, but using the above intermediate 9A instead of 2-fluoro-5-methylaniline. Yield 16.5%.
¹H-NMR (CDCl₃, δ): 2.01 (s, 1H, NH), 2.91-3.10 (m, 8H, piperazine CHs), 6.58-6.76 (m, 2H, phenyl H4 and H6), 7.19-7.31 (m, 1H, phenyl H3).

### 8-{2-[4-(2-Chloro-5-fluorophenyl)-1-piperazinyl]-ethyl}-8-azaspiro[4.5]decane-7,9-dione

The title compound was prepared according to the method used in Example 1, but using the above intermediate 9B instead of 1-(2,5-dichlorophenyl)-piperazine. Yield 74%, m.p. 139°C.
¹H-NMR (CDCl₃, δ): 1.43-1.57 (m, 4H, CH₂s of spiro ring), 1.69-1.80 (m, 4H, CH₂s of spiro ring), 2.57 (t, 2H, CONCH₂CH₂N), 2.60 (s, 4H, 2 CH₂CO), 2.63-2.77 (m, 4H, piperazine CHs), 2.87-3.09 (m, 4H, piperazine CHs), 3.97 (t, 2H, CONCH₂CH₂N), 6.58-6.80 (m, 2H, phenyl H4 and H6), 7.22-7.31 (m, 1H, phenyl H3).

### Example 10

### 8-{2-[4-(2-Chloro-5-iodophenyl)-1-piperazinyl]-ethyl}-8-azaspiro[4.5]decane-7,9-dione

### 1-(2-Chloro-5-iodophenyl)-piperazine (10A)

A solution of 0.32 g (2 mmol) of iodine monochloride in 5 ml of AcOH was added dropwise to a solution of 0.27 g (1 mmol) of 1-(2-chlorophenyl)-piperazine · 2HCl in 5 ml of AcOH. After 2 hours' stirring at room temperature, 5 ml of H₂O was added and the mixture stirred at 80°C for 2 hours. The solvents were evaporated, the residue dissolved in CH₂Cl₂, washed with Na₂S₂O₃ then with 10% KOH; the organic layer was dried (Na₂SO₄) and the solvent evaporated to dryness. The crude was purified by flash chromatography eluting with dichloromethane:2N-methanolic-ammonia 96:4 to give 0.032 g (10%) of the desired compound.
¹H-NMR (CDCl₃, δ): 1.79 (s, 1H, NH), 2.89-3.11 (m, 8H, piperazine CHs), 6.77 (d, 1H, phenyl H3), 7.49 (dd, 1H, phenyl H4), 7.63 (d, 1H, phenyl H6).

### 8-{2-[4-(2-Chloro-5-iodophenyl)-1-piperazinyl]-ethyl}-8-azaspiro[4.5]decane-7,9-dione

The title compound was prepared according to the method used in Example 1, but using the above intermediate 10A instead of 1-(2,5-dichlorophenyl)-piperazine, Yield 73%, m.p. 109-110°C.
¹H-NMR (CDCl₃, δ): 1.44-1.59 (m, 4H, CH₂s of spiro ring), 1.62-1.79 (m, 4H, CH₂s of spiro ring), 2.54 (t, 2H, CONCH₂CH₂N), 2.58 (s, 4H, 2 CH₂CO), 2.62-2.77 (m, 4H, piperazine CHs), 2.83-3.09 (m, 4H, piperazine CHs), 3.97 (t, 2H, CONCH₂CH₂N), 6.77 (d, 1H, phenyl H3), 7.49 (dd, 1H, phenyl H4), 7.64 (d, 1H, phenyl H6).

### Example 11

### 3-{2-[4-(2,5-Dichlorophenyl)-1-piperazinyl]-ethyl}-3-azaspiro[5.5]undecane-2,4-dione

### 3-(2-Bromoethyl)-3-azaspiro[5.5]undecane-2,4-dione (11A)

A suspension of 4.35 g (24 mmol) of 3-azaspiro[5.5]undecane-2,4-dione and 0.86 g (34.8 mmol) of 80% NaH in 48 ml of DMF was stirred at 60°C for 30 minutes. After cooling to room temperature, it was poured into a solution of 22.2 g (120 mmol) of 1,2-dibromoethane in 152 ml of DMF and the mixture was stirred for 5 hours. The solvent was evaporated in vacuo, the residue was treated with 200 ml of H₂O and extracted with CH₂Cl₂ (3 x 65 ml). The organic layer was washed with H₂O, dried (Na₂SO₄) and evaporated to dryness. The residue was purified by flash chromatography eluting with cyclohexane:ethyl acetate 7:3 to give 4.1 g (59%) of the desired compound. M.p. 65°C.
¹H-NMR (CDCl₃, δ): 1.18-1.60 (m, 10H, CH₂s azaspiro ring), 2.59 (s, 4H, 2 CH₂CO), 3.46 (t, 2H, NCH₂), 4.20 (t, 2H, CH₂Br).

### 3-{2-[4-(2,5-Dichlorophenyl)-1-piperazinyl)-ethyl}-3-azaspiro[5.5]undecane-2,4-dione

The title compound was prepared according to the method used in Example 1, but using the above intermediate 11A instead of 8-(2-bromoethyl)-8-azaspiro[4.5]decane-7,9-dione. Purification was carried out eluting with petroleum ether/ethyl acetate 85:15. Yield 66%.
¹H-NMR (CDCl₃, δ): 1.37-1.58 (m, 10H, CH₂s of spiro ring), 2.56 (s+t, 6H, 2 CH₂CO and CONCH₂CH₂N), 2.59-2.73 (m, 4H, piperazine CHs), 2.86-3.04 (m, 4H, piperazine CHs), 3.96 (t, 2H, CONCH₂CH₂N), 6.87 (d, 1H, phenyl H6), 6.96 (dd, 1H, phenyl H4), 7.23 (d, 1H, phenyl H3).

### Example 12

### 1-{2-[4-(2,5-Dichlorophenyl)-1-piperazinyl]-ethyl}-pyrrolidine-2,5-dione

### 2-[4-(2,5-dichlorophenyl)-1-piperazinyl]-acetonitrile (12A)

A mixture of 0.23 g (1 mmol) of 1-(2,5-dichlorophenyl)-piperazine, 0.15 g (2 mmol) of chloroacetonitrile, 0.21 g (1.5 mmol) of K₂CO₃ and 10 ml of toluene was stirred at reflux for 72 hours. The solvent was evaporated and the residue was treated with 10 ml of H₂O and extracted with ethyl acetate. After solvent evaporation, the crude was purified by flash chromatography eluting with petroleum ether/ethyl acetate 7:3 to give 0.15 g (55%) of the desired compound.
¹H-NMR (CDCl₃, δ): 2.74-2.87 (m, 4H, piperazine CHs), 3.10-3.22 (m, 4H, piperazine CHs), 3.59 (s, 2H, CH₂CN), 6.97 (dd, 2H, phenyl H4 and H6), 7.28 (d, 1H, phenyl H3).

### 2-[4-(2,5-dichlorophenyl)-1-piperazinyl]-ethylamine (12B)

A solution of 0.27 g (1 mmol) of the above intermediate 12A in 10 ml of anhydrous Et₂O was added dropwise to a stirred solution of 0.11 g (3 mmol) of lithium aluminium hydride in 10 ml of anhydrous Et₂O kept at 0°C. The mixture was stirred at room temperature for 1.5 hours, then cooled at -10/0°C and treated with 20% NaOH. The inorganic salts were filtered off and washed with CH₂Cl₂. The organic solutions were dried, then evaporated to dryness to give 0.24 g (90%) of the desired compound.
¹H-NMR (CDCl₃, δ): 1.48 (m, 2H, NH₂), 2.47 (t, 2H,CH₂NH₂), 2.56-2.75 (m, 4H, piperazine CHs), 2.78 (t, 2H, CH₂CH₂NH₂), 2.94-3.16 (m, 4H, piperazine CHs), 6.86 (d, 1H, phenyl H6), 6.96 (dd, 1H, phenyl H4), 7.23 (d, 1H, phenyl H3).

### 1-{2-[4-(2,5-Dichlorophenyl)-1-piperazinyl]-ethyl}-pyrrolidine-2,5-dione

A solution of 0.1 g (1 mmol) of succinic anhydride, 0.3 g (1.1 mmol) of the above intermediate 12B and 0.02 g of *p*-toluenesulphonic acid in 10 ml of toluene was stirred at reflux for 20 hours. After cooling to room temperature, the solvent was evaporated and the residue purified by flash chromatography eluting with toluene:acetone 9:1 to give 0.23 g (65%) of the title compound. M.p. 122°C.
¹H-NMR (CDCl₃, δ): 2.58 (t, 2H, CONCH₂CH₂N), 2.59-2.64 (m, 4H, piperazine CHs), 2.66 (s, 4H, 2 CH₂CO), 2.87-3.09 (m, 4H, piperazine CHs), 3.63 (t, 2H, CONCH₂CH₂N), 6.86 (d. 1H, phenyl H6), 6.96 (dd, 1H, phenyl H4), 7.22 (d, 1H, phenyl H3).

### Example 13

### 1-{2-[4-(2,5-Dichlorophenyl)-1-piperazinyl]-ethyl}-4-ethyl-4-methylpiperidinyl-2,6-dione

The title compound was prepared according to the method of Example 12, but using 3-ethyl-3-methylglutaric anhydride instead of succinic anhydride. Purification was carried out eluting with toluene:acetone 95:5. Yield 59%; m.p. 98-99°C.
¹H-NMR (CDCl₃, δ): 0.86 (t, 3H, CH₃CH₂), 1.03 (s, 3H, CH₃C), 1.40 (q, 2H, CH₃CH₂), 2.47 (s, 4H, 2 CH₂CO), 2.56 (t, 2H, CONCH₂CH₂N), 2.59-2.73 (m, 4H, piperazine CHs), 2.83-3.10 (m, 4H, piperazine CHs), 3.94 (t, 2H, CONCH₂CH₂N), 6.88 (d, 1H, phenyl H6), 6.95 (dd, 1H, phenyl H4), 7.21 (d, 1H, phenyl H3).

### Example 14

### 2-{2-[4-(2,5-Dichlorophenyl)-1-piperazinyl]-ethyl}-2-azaspiro[4.4]nonane-1,3-dione

### Ethyl cyclopentanecarboxylate (14A)

0.15 g (1.2 mmol) of oxalyl chloride was added dropwise at 0°C to a stirred solution of 0.11 g (1 mmol) of cyclopentanecarboxylic acid in 10 ml of CH₂Cl₂ and 2 drops of DMF. The solution was then stirred at room temperature for 4 hours. The solvent was evaporated off, the residue dissolved in 10 ml of CH₂Cl₂ and this solution was added dropwise at 0°C to 10 ml of ethanol. After 20 hours' stirring at room temperature, the solvents were evaporated to dryness and the residue purified by flash chromatography eluting with petroleum ether:ethyl acetate 98:2 to give 0.042 g (32%) of the desired compound.
¹H-NMR (CDCl₃, δ): 1.27 (t, 3H, CH₂CH₃), 1.44-1.98 (m, 8H, cyclopentane CH₂s), 2.61-2.82 (m, 1H, CH), 4.11 (q, 2H, CH₂CH₃).

### Ethyl (1-ethoxycarbonyl-1-cyclopentyl)-acetate (14B)

0.4 ml (1 mmol) of 2.5 M *n*-butyl lithium in THF was added dropwise to a solution of 0.1 g (1 mmol) of diisopropylamine in 10 ml of anhydrous THF stirred at -70°C under a N₂ atmosphere. After 30 minutes stirring, a solution of 0.14 g (1 mmol) of the above intermediate 14A in 5 ml of anhydrous THF was added and stirring was continued for 1 hour, then a solution of 0.17 g (1 mmol) of ethyl bromoacetate in 5 ml of anhydrous THF was added. After 1 hour stirring at -70°C, the mixture was heated at room temperature and stirred for 20 hours, then a solution of 0.43 g of NH₄Cl in 20 ml of H₂O was added and the mixture extracted with CH₂Cl₂. The organic layer was washed with H₂O, dried (Na₂SO₄) and evaporated to dryness. The residue was purified by flash chromatography eluting with petroleum ether/ethyl acetate 95:5 to give 0.044 g (19%) of the desired compound.
¹H-NMR (CDCl₃, δ): 1.21 (t, 6H, CH₂CH₃), 1.44-1.93 (m, 6H, cyclopentyl CHs), 2.02-2.21 (m, 2H, cyclopentyl CHs), 2.67 (s, 2H, CH₂CO), 4.11 (q, 4H, CH₂CH₃).

### 2-{2-[4-(2,5-Dichlorophenyl)-1-piperazinyl]-ethyl}-2-azaspiro[4.4]nonane-1,3-dione

A mixture of 0.11 g (0.48 mmol) of the above intermediate 14B, 0.23 g (0.84 mmol) of intermediate 12B was heated overnight at 120°C in an autoclave. After cooling to room temperature, the crude was purified by flash chromatography eluting with toluene:acetone 9:1 to give 0.03 g (15%) of the title compound.
¹H-NMR (CDCl₃, δ): 1.60-1.82 (m, 4H, CH₂s of spiro ring), 1.83-2.24 (m, 4H, CH₂s of spiro ring), 2.57 (s, 2H, CH₂CO), 2.62-2.75 (m, 6H, CONCH₂CH₂N and piperazine CHs), 2.93-3.09 (m, 4H, piperazine CHs), 3.68 (t, 2H, CONCH₂CH₂N), 6.96 (dd, 2H, phenyl H4 and H6), 7.23 (d, 1H, phenyl H3).

### Example 15

### 7-{2-[4-(2,5-Dichlorophenyl)-1-piperazinyl]-ethyl}-7-azaspiro[3.5]nonane-6,8-dione

A solution of 0.13 g (0.75 mmol) of cyclobutane-1,1-diacetic acid, prepared as described in *Bull. Soc. Chim. Fr*. 2572-2581 (1964), and 0.17 g (0.83 mmol) of dicyclohexylcarbodiimide in 5 ml of anhydrous DMF was stirred at room temperature for 1.5 hours. Then a solution of 0.21 g (0.79 mmol) of intermediate 12B in 1 ml of anhydrous DMF was added and the mixture stirred for 18 hours. The precipitate was separated by filtration and the solvent evaporated to dryness. 0.18 g (2.26 mmol) of sodium acetate and 5 ml of acetic anhydride were added to the residue and the resulting mixture was heated at 100°C for 2 hours. After cooling to room temperature, the suspension was poured into 10 ml of iced H₂O, stirred for 30 min, alkalinized with 2.5 N NaOH, and extracted with CH₂Cl₂. The organic layer was washed with H₂O, dried (Na₂SO₄) and evaporated to dryness. The residue was purified by flash chromatography eluting with toluene:acetone 8:2 to give 0.09 g (29%) of the title compound.
¹H-NMR (CDCl₃, δ): 1.84-2.06 (m, 6H, CH₂s of spiro ring), 2.51 (t, 2H, CONCH₂CH₂N), 2.57-2.68 (m, 4H, piperazine CHs), 2.73 (s, 4H, 2 CH₂CO), 2.87-3.09 (m, 4H, piperazine CHs), 3.94 (t, 2H, CONCH₂CH₂N), 6.88 (dd, 1H, phenyl H4), 6.96 (d, 1H, phenyl H6), 7.23 (d, 1H, phenyl H3).

### Example 16

### 8-{2-[4-(2,5-Dichlorophenyl)-1-piperazinyl]-1-methylethyl}-8-azaspiro[4.5]decane-7,9-dione

### 8-(2-Chloro-1-methylethyl)-8-azaspiro[4.5]decane-7,9-dione (16A)

A solution of 0.17 g (1 mmol) of diethyl azodicarboxylate in 1 ml of DMF was added dropwise to a solution of 0.17 g (1 mmol) of 8-azaspiro[4.5]decane-7,9-dione, 0.19 g (2 mmol) of 1-chloro-2-propanol and 0.26 g (1 mmol) of triphenylphosphine in 3 ml of DMF, and the mixture was stirred at 35°C for 72 hours. After cooling to room temperature, 20 ml of H₂O was added and the mixture extracted with CH₂Cl₂. The organic layer was washed with H₂O, dried (Na₂SO₄) and evaporated to dryness. The residue was purified by flash chromatography eluting with petroleum ether/ethyl acetate 9:1 to give 0.067 g (28%) of the desired compound.
¹H-NMR (CDCl₃, δ): 1.29-1.41 (m, 3H, CH₃), 1.42-1.58 (m, 4H, CH₂s of spiro ring), 1.60-1.78 (m, 4H, CH₂s of spiro ring), 2.57 (s, 4H, 2 CH₂CO), 3.57-3.88 (m, 1H, CH(H)Cl), 4.03-4.19 (m, 1H, CH(H)Cl), 4.96-5.13 (m, 1H, NCH).

### 8-{2-[4-(2,5-Dichlorophenyl)-1-piperazinyl]-1-methylethyl}-8-azaspiro[4.5]decane-7,9-dione

The title compound was prepared according to the method of Example 1, but using the above intermediate 16A instead of 8-(2-bromoethyl)-8-azaspiro[4.5]decane-7,9-dione. Heating lasted 45 minutes and purification was carried out eluting with toluene/acetone 95:5. Yield 21%.
¹H-NMR (CDCl₃, δ): 1.34 (d, 3H, CH₃), 1.51-1.61 (m, 4H, CH₂s of spiro ring), 1.62-1.80 (m, 4H, CH₂s of spiro ring), 2.31-2.43 (m, 1H, CH(CH₃)CH(H)N), 2.44-2.55 (m, 2H, piperazine CHs), 2.58 (s, 4H, 2 CH₂CO), 2.62-2.77 (m, 2H, piperazine CHs), 2.85-3.05 (m, 4H, piperazine CHs), 3.06-3.22 (m, 1H, CH(CH₃)CH(H)N), 4.95-5.12 (m, 1H, NCH), 6.93 (dd, 2H, phenyl H4 and H6), 7.22 (d, 1H, phenyl H3).

### Example 17

### 8-{2-[4-(2,5-Dichlorophenyl)-1-piperidinyl]-ethyl}-8-azaspiro[4,5]decane-7,9-dione

### Diethyl 2,5-dichlorobenzylidenemalonate (17A)

A mixture of 0.17 g (1 mmol) of 2,5-dichlorobenzaldehyde, 0.32 g (2 mmol) of diethyl malonate and 0.66 g (5 mmol) of AlCl₃ was stirred at room temperature for 4 hours, then poured into ice-water mixture. 37% HCl was then added and the mixture extracted with CH₂Cl₂. The organic layer was dried (Na₂SO₄) and evaporated, and the residue purified by flash chromatography eluting with petroleum ether/ethyl acetate 9:1 to give 0.16 g (51%) of the desired compound.
¹H-NMR (CDCl₃, δ): 1.24 (t, 3H, CH₂CH₃), 1.37 (t, 3H, CH₂CH₃), 4.31 (q, 4H, 2 CH₂CH₃), 7.24 (dd, 2H, phenyl H3 and H4), 7.41 (d, 1H, phenyl H6), 7.94 (s, 1H, CH).

### Diethyl 3-(2,5-dichlorophenyl)-2,4-diethoxycarbonylglutarate (17B)

A mixture of 0.31 g (1 mmol) of the above intermediate 17A, 0.16 g (1 mmol) of diethyl malonate and 0.4 g (3 mmol) of AlCl₃ was heated at 60°C for 6 hours. Isolation of the crude and its purification was carried out as for intermediate 17A and gave 0.16 g (34%) of the desired compound.
¹H-NMR (CDCl₃, δ): 1.07 (t, 6H, 2 CH₂CH₃), 1.22 (t, 6H, 2 CH₂CH₃), 4.01 (q, 4H, 2 CH₂CH₃), 4.12 (q, 6H, 2 CH₂CH₃ and 2 CHCO), 4.68-4.86 (m, 1H, CHCHCH), 7.10 (dd, 1H, phenyl H4), 7.22 (d, 1H, phenyl H3), 7.46 (d, 1H, phenyl H6).

### 3-(2,5-Dichlorophenyl)-glutaric acid (17C)

A mixture of 0.48 g (1 mmol) of the above intermediate 17B and 5 ml of 47% HBr was heated at reflux for 48 hours. After cooling to room temperature, the precipitate was collected by suction and dried to give 0.2 g (73%) of the desired compound.
¹H-NMR (DMSO-d₆, δ): 2.40 (t, 4H, 2 CH₂COO), 3.96 (q, 1H, CH), 7.31 (dd, 1H, phenyl H4), 7.49 (d, 1H, phenyl H3), 7.57 (d, 1H, phenyl H6), 12.50 (s, 2H, 2 COOH).

### 1-Cyanomethyl-4-(2,5-dichlorophenyl)-piperidine-2,6-dione (17D)

A solution of 0.28 g (1 mmol) of the above intermediate 17C and 0.23 g (1.1 mmol) of N,N'-dicyclohexylcarbodiimide in 5 ml of anhydrous DMF was stirred at room temperature for 1.5 hours, then 0.22 g (1.05 mmol) of aminoacetonitrile and 0.11 g (1.05 mmol) of triethylamine were added. The mixture was stirred at room temperature for 18 hours, then the precipitate was filtered off and the solvent evaporated under vacuum. 0.25 g (3 mmol) of sodium acetate and 5 ml of acetic anhydride were added to the residue and the mixture was heated at 100°C for 2 hours. After cooling to room temperature, the suspension was poured into 10 ml of ice water, stirred for 30 minutes, alkalinized with 2.5 N NaOH and extracted with CH₂Cl₂. The organic layer was dried (Na₂SO₄) and evaporated to dryness. The residue was purified by flash chromatography eluting with petroleum ether/ethyl acetate 85:15 to give 0.14 g (47%) of the desired compound.
¹H-NMR (CDCl₃, δ): 2.72-2.96 (m, 2H, CH₂CO), 3.01-3.22 (m, 2H, CH₂CO), 3.71 (s, 2H, CH₂CN), 3.73-3.97 (m, 1H, CH), 7.24 (dd, 1H, phenyl H4), 7.38 (d, 1H, phenyl H3), 7.67 (d, 1H, phenyl H6).

### 2-[4-(2,5-dichlorophenyl)-1-piperidinyl]-ethylamine (17E)

A solution of 0.3 g (1 mmol) of the above intermediate 17D in 5 ml of anhydrous Et₂O was added dropwise to a solution of 0.15 g (4 mmol) of LiAlH₄ in 5 ml of anhydrous Et₂O maintaining a gentle reflux. The mixture was stirred at reflux for 2 hours, then rested overnight at room temperature. The mixture was cooled at -10/0°C and the excess of LiAlH₄ was neutralised by 20% NaOH addition. The inorganic salts were filtered and washed with CH₂Cl₂. The organic layer was dried (Na₂SO₄) and evaporated to dryness. The residue was purified by flash chromatography eluting with CH₂Cl₂:2N-methanolic-ammonia 98:2 to give 0.1 g (37%) of the desired compound.
¹H-NMR (CDCl₃, δ): 1.77-1.96 (m, 4H, 2 piperidine CHs and NH₂), 1.97-2.28 (m, 2H, piperidine CHs), 2.42-2.56 (m, 2H, NCH₂CH₂NH₂), 2.77-2.94 (m, 5H, piperidine CHs and NCH₂CH₂NH₂), 2.96-3.21 (m, 2H, piperidine CHs), 7.08-7.37 (m, 3H, phenyl CHs).

### 8-{2-[4-(2,5-Dichlorophenyl)-1-piperidinyl]-ethyl}-8-azaspiro[4.5]decane-7,9-dione

The title compound was prepared according to the method of Example 12, but using the above intermediate 17E instead of intermediate 12B and 3,3-tetramethyleneglutaric acid instead of succinic anhydride. Yield 27%.
¹H-NMR (CDCl₃, δ): 1.45-1.63 (m, 4H, CH₂s of spiro ring), 1.64-1.97 (m, 8H, CH₂s of spiro ring and 4 piperidine CHs), 2.07-2.23 (m, 2H, piperidine CHs), 2.50 (t, 2H, CONCH₂CH₂N), 2.64 (s, 4H, 2 CH₂CO), 2.83-3.16 (m, 3H, piperidine CHs), 4.38 (t, 2H, CONCH₂CH₂N), 7.01-7.24 (m, 3H, phenyl CHs).

### Example 18

### N-(2,5-Dichlorophenyl)-N'-{2-(7,9-dioxo-8-azaspiro[4.5]decane-8-yl)-ethyl}-N,N'-dimethyl-1,2-diaminoethane

### N-(2,5-Dichlorophenyl)-2-aminoethanol (18A)

A mixture of 0.16 g (1 mmol) of 2,5-dichloroaniline, 0.8 g (10 mmol) of 2-chloroethanol and 0.15 g (1.5 mmol) of triethylamine was stirred at 150°C for 16 hours. The crude was purified by flash chromatography eluting with cyclohexane:ethyl acetate 7:3 to give 0.09 g (45%) of the desired compound.
¹H-NMR (CDCl₃, δ): 1.59-1.88 (m, 1H, NH), 3.36 (t, 2H, NCH₂), 3.93 (t, 2H, CH₂O), 4.57-4.84 (m, 1H, OH), 6.63 (dd, 2H, phenyl H4 and H6), 7.17 (d, 1H, phenyl H3).

### N-(2,5-Dichlorophenyl)-2-methylamino-ethanol (18B)

A mixture of 0.21 g (1 mmol) of the above intermediate 18A, 0.083 ml (3 mmol) of 37% HCHO and 0.14 g (3 mmol) of formic acid was heated at reflux for 18 h. After cooling to room temperature, concentrated HCl was added until pH 1, the solvent evaporated to dryness, the residue alkalinized with 2 N NaOH, and extracted with CH₂Cl₂. The organic layer was dried (Na₂SO₄) and evaporated to dryness. The residue was purified by flash chromatography eluting with petroleum ether:ethyl acetate 8:2 to give 0.05 g (23%) of the desired compound.
¹H-NMR (CDCl₃, δ): 2.49-2.63 (m, 1H, OH), 2.77 (s, 3H, CH₃), 3.17 (t, 2H, NCH₂), 3.74 (t, 2H, CH₂O), 6.96 (dd, 2H, phenyl H4 and H6), 7.27 (d, 1H, phenyl H3).

### N-(2,5-Dichlorophenyl)-N-methyl-2-chloroethylamine (18C)

0.14 g (1.2 mmol) of thionyl chloride was added to a solution of 0.22 g (1 mmol) of the above intermediate 18B in 10 ml of CH₂Cl₂ containing 3 drops of DMF stirred at 0°C, and the mixture was stirred for 0.5 hours at room temperature and at reflux for 3 hours. The solvent was evaporated and the crude suspended in CH₂Cl₂ and re-evaporated. The residue was alkalinized with 2 N NaOH and extracted with CH₂Cl₂. The organic layer was dried (Na₂SO₄) and evaporated to dryness to give 0.19 g (83%) of the desired compound.
¹H-NMR (CDCl₃, δ): 2.85 (s, 3H, CH₃), 3.40 (t, 2H, NCH₂), 3.68 (t, 2H, CH₂Cl), 6.97 (dd, 1H, phenyl H4), 7.06 (d, 1H, phenyl H6), 7.24 (d, 1H, phenyl H3).

### N,N'-Dimethyl-N-(2,5-dichlorophenyl)-ethylenediamine (18D)

A mixture of 0.24 g (1 mmol) of the above intermediate 18C and 4.32 ml (8.65 mmol) of 2 N methanolic methylamine solution was heated at 60-80°C for 20 hours in an autoclave. After cooling to room temperature, the solvent was evaporated and the residue was purified by flash chromatography eluting with dichloromethane:2N-methanolic-ammonia 96:4 to give 0.067 g (29%) of the desired compound.
¹H-NMR (CDCl₃, δ): 1.93-2.02 (m, 1H, NH), 2.46 (s, 3H, CH₃), 2.77 (t+s, 5H, CH₂N and CH₃), 3.17 (t, 2H, NCH₂), 6.97 (dd, 1H, phenyl H4), 7.06 (d, 1H, phenyl H6), 7.24 (d, 1H, phenyl H3).

### N-(2,5-dichlorophenyl)-N'-{2-(7,9-dioxo-8-azaspiro[4.5]decane-8-yl)-ethyl}-N,N'-dimethyl-1,2-diaminoethane

The title compound was prepared according to the method used in Example 1, but using the above intermediate 18D instead of 1-(2,5-dichlorophenyl)-piperazine and heating at 140°C. Purification was carried out eluting with toluene/methanol 95:5. The residue was dissolved in dichloromethane, the solution acidified by ethereal HCl addition. The filtered hydrochloride salt was dissolved in dichloromethane, and the mixture made alkaline by NaOH addition. The organic layer was dried (Na₂SO₄) and evaporated to dryness to give the title compound. Yield 18%.
¹H-NMR (CDCl₃, δ): 1.43-1.59 (m, 4H, CH₂s of spiro ring), 1.61-1.79 (m, 4H, CH₂s of spiro ring), 2.36 (s, 3H, NCH₃), 2.44-2.73 (m, 8H, 2 CH₂CO and CONCH₂CH₂N(CH₃)CH₂), 2.82 (s, 3H, NCH₃), 3.09-3.18 (m, 2H, CH₂N(CH₃)Ar), 3.88 (t, 2H, CONCH₂CH₂), 6.90 (dd, 1H, phenyl H4), 7.01 (d, 1H, phenyl H6), 7.24 (d, 1H, phenyl H3).

### Example 19

### 8-{2-[4-(2,5-Dichlorophenyl)-1-piperazinyl]-ethyl}-8-azaspiro[4.5]decane-7-one

### Methyl (1-formylmethyl-1-cyclopentyl)-acetate (19A)

0.7 g (5.5 mmol) of oxalyl chloride was dropped into a solution of 1 g (5 mmol) of 1,1-cyclopentanediacetic acid monomethyl ester, prepared as described in *J. Chem*. *Soc*. 713-721 (1929), in 20 ml of CH₂Cl₂ containing some drops of DMF, at 10-15°C. After 2 hours' stirring at room temperature the solvent was evaporated to dryness. The residue was dissolved in 20 ml of acetone and this solution was added to a mixture of 2 ml of diisopropylethylamine and 0.1 g of 10% Pd/C in 20 ml of anhydrous acetone previously stirred in hydrogen atmosphere. The mixture was hydrogenated at normal pressure until one equivalent of hydrogen was adsorbed, then the catalyst was filtered off and the solvent evaporated to dryness. The residue was purified by flash chromatography eluting with cyclohexane:ethyl acetate 9:1 to give 0.63 g (69%) of the desired compound.
¹H-NMR (CDCl₃), δ): 1.39-1.63 (m, 8H, cyclopentyl CH₂s), 2.41 (s, 2H, CH₂COO), 2.58 (m, 2H, CH₂CHO), 3.57 (s, 3H, CH₃), 9.61-9.75 (m, 1H, CHO).

### 8-{2-[4-(2,5-Dichlorophenyl)-1-piperazinyl]-ethyl}-8-azaspiro[4.5]decane-7-one

0.009 g (0.29 mmol) of NaBH₄ was added to a stirred solution of 0.092 g (0.5 mmol) of the above intermediate 19A and 0.16 g (0.58 mmol) of intermediate 12B in 10 ml of MeOH, at 0°C. After 1.5 hours' stirring, the solvent was evaporated, the residue treated with 5 ml of H₂O and extracted with CH₂Cl₂ (3 x 10 ml). The organic layer was dried (Na₂SO₄) and evaporated to dryness. The residue was purified by flash chromatography eluting with toluene/acetone 75:25 to give 0.14 g (68%) of the title compound. M.p. 88-89°C.
¹H-NMR (CDCl₃, δ): 1.19-1.58 (m, 4H, CH₂s of spiro ring), 1.59-1.80 (m, 6H, CH₂s of spiro ring and CCH₂CH₂N), 2.27 (s, 2H, CH₂CO), 2.59 (t, 2H, CONCH₂CH₂N), 2.62-2.78 (m, 4H, piperazine CHs), 2.97-3.17 (m, 4H, piperazine CHs), 3.21 (t, 2H, CCH₂CH₂N), 3.57 (t, 2H, CONCH₂CH₂N), 6.88-7.02 (m, 2H, phenyl CHs), 7.22-7.32 (m, 1H, phenyl CH).
The monohydrochloride was also prepared, m.p. > 230°C (EtOH).

### Example 20

### 8-{2-[4-(2,5-Dichlorophenyl)-1-piperazinyl]-ethyl}-8-azaspiro[4.5]dec-9,10-en-7-one

A mixture of 0.08 g (0.44 mmol) of intermediate 19A, 0.14 g (0.51 mmol) of intermediate 12B, 0.02 g of *p*-toluenesulphonic acid and 10 ml of toluene was stirred at reflux overnight distilling off the azeotrope. After cooling to room temperature, the solvent was evaporated to dryness and the residue purified by flash chromatography eluting with toluene:acetone 9:1 to give 0.09 g (51%) of the title compound.
¹H-NMR (CDCl₃, δ): 1.43-1.59 (m, 4H, CH₂s of spiro ring), 1.61-1.77 (m, 4H, CH₂s of spiro ring), 2.38 (s, 2H, CH₂CO), 2.58 (t, 2H, CONCH₂CH₂), 2.60-2.98 (m, 4H, piperazine CHs), 2.96-3.15 (m, 4H, piperazine CHs), 3.61 (t, 2H, CONCH₂CH₂), 5.09 (d, 1H, H10), 5.96 (d, 1H, H9), 6.86-7.03 (m, 2H, phenyl CHs), 7.20-7.27 (m, 1H, phenyl CH).

### Example 21

### 8-{2-[4-(2,5-Dichlorophenyl)-1-piperazinyl]-ethyl}-8-azaspiro[4.5]decane

0.2 ml (1.62 mmol) of boron trifluoride etherate was added to a suspension of 0.15 g (0.33 mmol) of the compound from Example 19,0.05 g (1.32 mmol) of NaBH₄ in 10 ml of THF stirred at 0°C in nitrogen atmosphere. The mixture was stirred at room temperature for 4 hours and at 60°C for 5 hours. 4 ml of 2N HCl was then added, the solvent evaporated and the residue alkalinized with 1N NaOH up to pH 8. The mixture was extracted with dichloromethane (3 x 10 ml), the organic phase dried (Na₂SO₄) and the solvent evaporated to dryness. The residue was purified by flash chromatography eluting with toluene:acetone 95:5 to give 0.068 g of a crude which was heated at reflux for 2 hours in 10 ml of methanol and 0.5 ml of 2N HCl. After cooling to 20-25°C, the solvents were evaporated to dryness, the residue was alkalinized with 1N NaOH and extracted with dichloromethane (3 x 10 ml). The organic phase was dried (Na₂SO₄) and evaporated to dryness to give 0.058 g (44%) of the title compound as an oil.
¹H-NMR (CDCl₃, δ): 1.32-1.46 (m, 4H, CH₂s of spiro ring), 1,47-1,54 (m, 4H, CH₂s of spiro ring), 1.57-1.68 (m, 4H, CH₂s of spiro ring), 2.38-2.50 (m, 4H, CH₂s of spiro ring and CH₂s of ethyl chain), 2.51-2.63 (m, 4H, CH₂s of spiro ring and CH₂s of ethyl chain), 2.64-2.77 (m, 4H, piperazine CHs), 3.00-3.12 (m, 4H, piperazine CHs), 6.91 (d, 1H, phenyl CH), 6.99 (dd, 1H, phenyl CH), 7.27 (d, 1H, phenyl CH).

### Example 22

### 8-{2-[4-(2-Chloro-5-nitrophenyl)-1-piperazinyl]-ethyl}-8-azaspiro[4.5]decane-7,9-dione

### 1-(2-Chloro-5-nitrophenyl)-piperazine (22A)

The title compound was prepared according to the method described in Example 4 for intermediate 4A, but using 2-chloro-5-nitroaniline instead of 2-fluoro-5-methylaniline. Yield 6%.
¹H-NMR (CDCl₃, δ): 2.58 (s, 1H, NH), 2.92-3.14 (m, 8H, piperazine CHs), 7.42 (d, 1H, phenyl H3), 7.76 (dd, 2H, phenyl H4 and H6).

### 8-{2-[4-(2-Chloro-5-nitrophenyl)-1-piperazinyl]-ethyl}-8-azaspiro[4.5]decane-7,9-dione

The title compound was prepared according to the method described in Example 4, but using the above intermediate 22A instead of intermediate 4A. Yield 31%; m.p. 116-117°C.
¹H-NMR (CDCl₃, δ): 1.46-1.61 (m, 4H, CH₂s of spiro ring), 1.62-1.79 (m, 4H, CH₂s of spiro ring), 2.57 (t, 2H, CONCH₂CH₂N), 2.59 (s, 4H, 2 CH₂CO), 2.64-2.77 (m, 4H, piperazine CHs), 2.98-3.17 (m, 4H, piperazine CHs), 3.97 (t, 2H, CONCH₂CH₂N), 7.24 (d, 1H, phenyl H3), 7.78 (dd, 2H, phenyl H4 and H6).

### Example 23

### 8-{2-[4-(2,5-Dichlorophenyl)-1-piperazinyl]-ethyl}-8-azaspiro[4.5]decane-7-one N¹-oxide

A solution of 0.3 g (0.615 mmol) of magnesium monoperoxyphthalate hexahydrate in 4 ml of H₂O was dropped at 10-15°C to a stirred solution of 0.5 g (1.23 mmol) of the compound of Example 19 in 10 ml of methanol and 10 ml of chloroform. The mixture was stirred at 20-25°C for 4 hours then the solvent was evaporated to dryness. The residue was treated with 10 ml of H₂O, alkalinized with a 20% sodium carbonate aqueous solution until pH 8 and extracted with dichloromethane (3 x 5 ml). The organic layer was dried (Na₂SO₄), filtered and evaporated to dryness. The residue was purified by flash chromatography eluting with dichloromethane:methanol 9:1 to afford 0.41 g (78%) of the title compound. M.p. 153.9-154.3°C.
¹H-NMR (CDCl₃, δ): 1.40-1.59 (m, 4H, CH₂s of spiro ring), 1.60-1.82 (m, 6H, CH₂s of spiro ring), 2.25 (s, 2H, CH₂CO), 3.20-3.35 (m, 2H, piperazine CHs), 3.49-3.62 (m, 4H, piperazine CHs), 3.62-3.79 (m, 6H, CONCH₂CH₂C, CONCH₂CH₂N and 2 piperazine CHs), 3.90-4.06 (m, 2H, CONCH₂CH₂N), 7.06 (dd, 2H, phenyl CHs), 7.31 (d, 1H, phenyl CH).

### Example 24

### 8-{2-[4-(2,4,5-Trifluorophenyl)-1-piperazinyl]-ethyl}-8-azaspiro[4.5]decane-7-one

### 1-(2,4,5-Trifluorophenyl)-piperazine (24A)

A mixture of 0.74 g (5mmol) of 2,4,5-trifluoroaniline, 0.91 g (5 mmol) of *bis*-(2-chloroethyl)amine hydrochloride, 2.5 ml of o-dichlorobenzene, and 0.25 ml of n-hexanol was stirred at reflux temperature for 7.5 hours. After cooling to room temperature, the mixture was treated with 2N NaOH (10 ml) and extracted with diethyl ether (3 x 20 ml). Purification was carried out by flash chromatography eluting with dichloromethane:2N methanolic ammonia gradient from 100:5 to 100:10 to give 0.65 g of the title compound. Yield: 60%.
¹H-NMR (CDCl₃, δ): 2.97-3.18 (m, 8H, piperazine CHs), 3.32 (s, 1H, NH); 6.68-7.00 (m, 2H, phenyl CHs).

### 2-[4-(2,4,5-trifluorophenyl)-1-piperazinyl]-acetonitrile (24B)

This compound was prepared according to the method of Example 12 for compound 12A, but using the above intermediate 24A instead of 1-(2,5-dichlorophenyl)-piperazine, and the mixture was stirred at 140°C for 5 hours. Yield 59%.
¹H-NMR (CDCl₃, δ): 2.72-2.86 (m, 4H, piperazine CHs), 3.05-3.19 (m, 4H, piperazine CHs), 3.56 (s, 2H, CH₂CN), 6.69-6.81 (m, 1H, phenyl. CH), 6.82-7.01 (m, 1H, phenyl CH).

### 2-[4-(2,4,5-trifluorophenyl)-1-piperazinyl]-ethylamine (24C)

This compound was prepared according to the method of Example 12 for compound 12B, but using the above intermediate 24B instead of intermediate 12A. Yield 93%.
¹H-NMR (CDCl₃, δ): 1.50 (s, 2H, NH₂), 2.51 (t, 2H, CH₂NH₂), 2.56-2.74 (m, 4H, piperazine CHs), 2.83 (t, 2H, CH₂CH₂NH₂), 2.96-3.17 (m, 4H, piperazine CHs), 6.68-7.01 (m, 2H, phenyl CHs).

### 8-{2-[4-(2,4,5-Trifluorophenyl)-1-piperazinyl]-ethyl}-8-azaspiro[4.5]decane-7-one

The title compound was prepared according to the method of Example 19, but using the above intermediate 24C instead of intermediate 12B. Yield 74%; m.p. 98.5-99°C.
¹H-NMR (CDCl₃, δ): 1.41-1.57 (m, 4H, CH₂s of spiro ring), 1.58-1.79 (m, 6H, CH₂s of spiro ring), 2.37 (s, 2H, CH₂CO), 2.61 (t, 2H, CONCH₂CH₂N), 2.63-2.79 (m, 4H, piperazine CHs), 2.96-3.12 (m, 4H, piperazine CHs), 3.41 (t, 2H, CONCH₂CH₂C), 3.56 (t, 2H, CONCH₂CH₂N), 6.68-7.01 (m, 2H, phenyl CHs).

### Example 25

### 3-{2-[4-(2,5-Dichlorophenyl)-1-piperazinyl]-emyl}-3-azaspiro[5.5]undecane-2-one

### Methyl (1-formylmethyl-1-cyclohexyl)-acetate (25A)

This compound was prepared according to the method of Example 19 for compound 19A, but using 1,1-cyclohexanediacetic acid monomethyl ester, prepared as described in *J. Chem. Soc.* 713-721 (1929), instead of 1,1-cyclopentanediacetic acid monomethyl ester. Purification was carried out by flash chromatography eluting with petroleum ether:ethyl acetate 95:5. Yield 39%.
¹H-NMR (CDCl₃, δ): 1.38-1.59 (m, 10H, cyclohexane CH₂s), 2.51 (s, 2H, CH₂COOCH₃), 2.59 (d, 2H, CH₂CHO), 3.66 (s, 3H, COOCH₃), 9.87 (t, 1H, CHO).

### 3-{2-[4-(2,5-Dichlorophenyl)-1-piperazinyl]-ethyl}-3-azaspiro[5.5]undecane-2-one

The title compound was prepared according to the method of Example 19, but using the above intermediate 25A instead of 19A. Purification was carried out by flash chromatography eluting with toluene:acetone 8:2.Yield 51%; m.p. 65.5-66°C.
¹H-NMR (CDCl₃, δ): 1.32-1.55 (m, 10H, CH₂s of spiro ring), 1.70 (t, 2H, CONCH₂CH₂C), 2.25 (s, 2H, CH₂CO), 2.58-2.75 (m, 6H, piperazine CHs and CONCH₂CH₂N), 2.97-3.10 (m, 4H, piperazine CHs), 3.38 (t, 2H, CONCH₂CH₂C), 3.52 (t, 2H, CONCH₂CH₂N), 6.89-7.00 (m, 2H, phenyl CHs), 7.25 (d, 1H, phenyl CH).

### Example 26

### 3-{2-[4-(2,5-Dichlorophenyl)-1-piperazinyl]-ethyl}-3-azaspiro[5.6]dodecane-2,4-dione.

The title compound was prepared according to the method of Example 15, using cycloheptane-1,1-diacetic acid, prepared as described in *Indian J. Chem.* 1, 256-258 (1963), instead of cyclobutane-1,1-diacetic acid. The residue was purified by flash chromatography eluting with toluene:acetone 95:5. Yield 36%.
¹H-NMR (CDCl₃, δ): 1.36-1.61 (m, 12H, CH₂s of spiro ring), 2.51-2.61 (m, 6H, 2 CH₂CO and CONCH₂CH₂N), 2.63-2.74 (m, 4H, piperazine CHs), 2.89-3.08 (m, 4H, piperazine CHs), 3.97 (t, 2H, CONCH₂CH₂N), 6.96 (dd, 2H, phenyl CHs), 7.27 (d, 1H, phenyl CHs).

### Example 27

### 8-{2-[4-(5-Chloro-2-cyanophenyl)-1-piperazinyl]-ethyl}-8-azaspiro[4.5]decane-7-one

### 8-(2-Hydroxyethyl)-8-azaspiro[4.5]decane-7-one (27A)

To a solution of 1.1 g (6 mmol) of intermediate 19A and 0.42 ml (7.02 mmol) of 2-aminoethanol, cooled at 0°C, 0.11 g (3 mmol) of NaBH₄ was added and the resulting mixture stirred at 20-25°C for 45 minutes. The solvent was evaporated to dryness at reduced pressure, H₂O (25 ml) was added and the solution extracted with CH₂Cl₂ (3x20 ml). The combined organic layers were dried over Na₂SO₄, filtered and evaporated *in vacuo*. The crude was purified by flash chromatography eluting with ethyl acetate:methanol 95:5 to give 0.87 g (76%) of the title compound as an oil.
¹H-NMR (CDCl₃, δ): 1.41-1.59 (m, 4H, CH₂s of spiro ring), 1.61-1.72 (m, 4H, CH₂s of spiro ring), 1.74 (t, 2H, CONCH₂CH₂C), 2.31 (s, 2H, CH₂CO), 2.60 (bs, 1H, OH), 3.02 (t, 2H, CONCH₂CH₂OH), 3.39 (t, 2H, CONCH₂CH₂C), 3.79 (t, 2H, CONCH₂CH₂OH).

### 8-(2-Chloroethyl)-8-azaspiro[4.5]decane-7-one (27B)

To a solution of 0.28 g (1.4 mmol) of the above intermediate 27A in 10 ml of CH₂Cl₂ cooled at 0°C, a solution of 0.12 ml (1.69 mmol) of SOCl₂ in 2 ml of CH₂Cl₂ and DMF (5 drops) was added and the mixture stirred at 20-25°C for 30 minutes, then refluxed for 3 hours. After cooling to room temperature, the solvent was evaporated to dryness, CH₂Cl₂ (20 ml) was added and evaporated. The crude was alkalinized with 1 N NaOH (pH=8) and extracted with CH₂Cl₂ (3x10 ml). The combined organic layers were dried over Na₂SO₄, filtered and evaporated to dryness under vacuum to give 0.29 g (95%) of the desired compound as an oil.
¹H-NMR (CDCl₃, δ): 1.38-1.53 (m, 4H, CH₂s of spiro ring), 1.61-1.74 (m, 4H, CH₂s of spiro ring), 1.75 (t, 2H, CONCH₂CH₂C), 2.32 (s, 2H, CH₂CO), 3.47 (t, 2H, CONCH₂CH₂C), 3.61-3.79 (m, 4H, CH₂CH₂Cl).

### 8-{2-[4-(5-Chloro-2-cyanophenyl)-1-piperazinyl]-ethyl}-8-azaspiro[4.5]decane-7-one

The title compound was prepared according to the method used in Example 1, but using intermediate 8A instead of 1-(2,5-dichlorophenyl)-piperazine and the above intermediate 27B instead of 8-(2-bromoethyl)-8-azaspiro[4.5]decane-7,9-dione. The crude was purified by flash chromatography eluting with petroleum ether/ethyl acetate gradient from 2:8 to 0:10. Yield 33% of the title compound as an oil.
¹H-NMR (CDCl₃, δ): 1.39-1.51 (m, 4H, CH₂s of spiro ring), 1.54-1.72 (m, 4H, CH₂s of spiro ring), 1.75 (t, 2H, CONCH₂CH₂C), 2.24 (s, 2H, CH₂CO), 2.69 (t, 2H, CONCH₂CH₂N), 2.70-2.88 (m, 4H, piperazine CHs), 3.21-3.32 (m, 4H, piperazine CHs), 3.39 (t, 2H, CONCH₂CH₂C), 3.54 (t, 2H, CONCH₂CH₂N), 6.97 (dd, 2H, phenyl H4 and H6), 7.48 (dd, 1H, phenyl H3).

### Example 28

### 8-{2-[4-(5-Chloro-2-fluorophenyl)-1-piperazinyl]-ethyl}-8-azaspiro[4.5]decane-7-one

### 1-(5-Chloro-2-fluorophenyl)-piperazine (28A)

A mixture of 1 g (6.87 mmol) of 5-chloro-2-fluoroaniline, 1.35 g (7.55 mmol) of *bis-(2*-chloroethyl)-amine hydrochloride, 3 ml of *o*-dichlorobenzene and 0.3 ml of *n*-hexanol was stirred at reflux temperature for 11 h. After cooling to room temperature, the mixture was treated with 10 ml of 2 N NaOH and extracted with CH₂Cl₂ (3x20 ml). The combined organic layers were washed with H₂O, dried (Na₂SO₄) and evaporated to dryness *in vacuo.* The purification was carried out by flash chromatography eluting with CH₂Cl₂ : 2 N methanolic ammonia 97:3 to give 1.13 g (76.6%) of the title compound.
¹H-NMR (CDCl₃, δ): 2.06 (bs, 1H, NH), 3.01-3.12 (m, 8H, piperazine CHs), 6.86-7.03 (m, 3H, phenyl CHs).

### N-{2-[4-(5-Chloro-2-fluorophenyl)-1-piperazinyl]-ethyl}-phthalimide (28B)

A mixture of 1.13 g (5.26 mmol) of the above intermediate 28A, 1.31 g (5.16 mmol) of 1-(2-bromoethyl)-phthalimide and 1.82 g (13.15 mmol) of K₂CO₃ in 20 ml of CH₃CN was stirred under N₂ atmosphere at 20-25°C for 6 h, then at 50-60°C for 9 h. After cooling to room temperature, the solvent was evaporated at reduced pressure, H₂O (20 ml) was added and the mixture extracted with CH₂Cl₂ (3x15 ml). The combined organic layers were dried over Na₂SO₄, filtered and evaporated to dryness. The crude was purified by flash chromatography eluting with CH₂Cl₂ : EtOAc 95:5 affording 0.99 g (48%) of the title compound.
¹H-NMR (CDCl₃, δ): 2.58-2.79 (m, 6H, piperazine CHs and CONCH₂CH₂N), 2.92-3.08 (m, 4H, piperazine CHs), 3.85 (t, 2H, CONCH₂CH₂N), 6.79-6.99 (m, 3H, phenyl CHs), 7.63-7.77 (m, 2H, phthalimide CHs), 7.81-7.89 (m, 2H, phthalimide CHs).

### 2-[4-(5-Chloro-2-fluorophenyl)-1-piperazinyl]-ethylamine (28C)

A solution of 0.99 g (2.57 mmol) of the above intermediate 28B and 0.37 ml ( 7.70 mmol) of NH₂NH₂ H₂O in 40 ml of EtOH was refluxed for 3 h. After cooling to room temperature, the precipitate was filtered off, washed with iced EtOH (30 ml), then with Et₂O (40 ml). The solution was alkalinised with 1 N NaOH (pH=8) and extracted with CH₂Cl₂ (3x15 ml). The organic phase was dried (Na₂SO₄), filtered and evaporated to dryness at reduced pressure to give 0.67 g (100%) of the desired compound as an oil.
¹H-NMR (CDCl₃, δ): 1.55 (bs, 2H, NH₂), 2.50 (t, 2H, NCH₂CH₂NH₂), 2.57-2.61 (m, 4H, piperazine CHs), 2.81 (t, 2H, NCH₂CH₂NH₂), 3.08-3.17 (m, 4H, piperazine CHs), 6.83-7.02 (m, 3H, phenyl CHs).

### 8-{2-[4-(5-Chloro-2-fluorophenyl)-1-piperazinyl]-ethyl}-8-azaspiro[4.5]decane-7-one

The title compound was prepared according to the method of Example 19, using intermediate 28C instead of intermediate 12B. Purification was carried out eluting with toluene / acetone gradient from 9:1 to 8.5:1.5. Yield 66%; m.p. 82-83 °C.
¹H-NMR (CDCl₃, δ): 1.48-1.53 (m, 4H, CHs of spiro ring), 1.59-1.78 (m, 4H, CHs of spiro ring), 1.72 (t, 2H, CCH₂CH₂NCO), 2.25 (s, 2H, CH₂CO), 2.51-2.77 (m, 6H, piperazine CHs and CONCH₂CH₂N), 2.98-3.17 (m, 4H, piperazine CHs), 3.38 (t, 2H, CCH₂CH₂NCO), 3.53 (t, 2H, CONCH₂CH₂N), 6.81-7.01 (m, 3H, phenyl CHs).

### Example 29

### 8-{2-[4-(5-Chloro-2-methylphenyl)-1-piperazinyl]-ethyl}-8-azaspiro[4.5]decane-7-one

### 1-(5-Chloro-2-methylphenyl)-piperazine (29A)

This compound was prepared according to the method described in Example 4 for intermediate 4A, but using 5-chloro-2-methylaniline instead of 2-fluoro-5-methylaniline. Yield 47%.
¹H-NMR (CDCl₃, δ): 1.91 (s, 1H; NH), 2.24 (s, 3H, CH₃), 2.77-2.95 (m, 4H, piperazine CHs), 2.96-3.09 (m, 4H, piperazine CHs), 6.96 (dd, 2H, phenyl H4 and H6), 7.09 (d, 1H, phenyl H3).

### N-{2-[4-(5-Chloro-2-methylphenyl)-1-piperazinyl]-ethyl}-phthalimide (29B)

This compound was prepared according to the method described in Example 28 for intermediate 28B, but using intermediate 29A instead of intermediate 28A. Yield 26.5%.
¹H-NMR (CDCl₃, δ): 2.23 (s, 3H, CH₃), 2.61-2.79 (m, 6H, piperazine CHs and CONCH₂CH₂N), 2.81-2.98 (m, 4H, piperazine CHs), 3.89 (t, 2H, CONCH₂CH₂N), 6.96 (dd, 2H, phenyl H4, H6), 7.07 (d, 1H, phenyl H3), 7.69-7.79 (m, 2H, phthalimide CHs), 7.81-7.91 (m, 2H, phthalimide CHs).

### 2-[4-(5-Chloro-2-methylphenyl)-1-piperazinyl]-ethylamine (29C)

This compound was prepared according to the method described in Example 28 for intermediate 28C, but using intermediate 29B instead of intermediate 28B. Yield 100%.
¹H-NMR (CDCl₃, δ): 1.61 (bs, 2H, NH₂), 2.23 (s, 3H, CH₃), 2.49 (t, 2H, NCH₂CH₂NH₂), 2.57-2.72 (m, 4H, piperazine CHs), 2.84 (t, 2H, NCH₂CH₂NH₂), 2.88-2.99 (m, 4H, piperazine CHs), 6.94 (dd, 2H, phenyl H4 and H6), 7.07 (d, 1H, phenyl H3).

### 8-{2-[4-(5-Chloro-2-methylphenyl)-1-piperazinyl]-ethyl}-8-azaspiro[4.5]decane-7-one

The title compound was prepared according to the method of Example 19, using the above intermediate 29C instead of intermediate 12B. Purification was carried out eluting with CH₂Cl₂ : EtOAc gradient from 1:1 to 4:6. Yield 62%; m.p. 94-95°C.
¹H-NMR (CDCl₃, δ): 1.42-1.52 (m, 4H, CH₂s of spiro ring), 1.66-1.74 (m, 4H, CH₂s of spiro ring), 1.80 (t, 2H, CCH₂CH₂NCO), 2.24 (s, 3H, CH₃), 2.27 (s, 2H, CH₂CO), 2.57-2.76 (m, 6H, piperazine CHs and CONCH₂CH₂N), 2.86-2.99 (m, 4H, piperazine CHs), 3.41 (t, 2H, CCH₂CH₂NCO), 3.52 (t, 2H, CONCH₂CH₂N), 6.96 (dd, 2H, phenyl H4 and H6), 7.07 (d, 1H, phenyl H3).

### Example 30

### Determination of affinity for cloned α₁-adrenergic and 5-HT_{1A}-serotoninergic receptors by radioligand binding assay

The determination of affinity for cloned subtypes of human α₁ adrenergic receptors was performed in membranes from CHO cells (chinese hamster ovary cells) transfected by electroporation with DNA expressing the genes encoding each α₁-adrenoceptor subtype, namely α₁ₐ, α_{1b} and α_{1d.}.
Cloning and stable expression of the human α₁-adrenoceptor gene were performed as previously described (Testa et al., Pharmacol. Comm. 6, 79-86 (1995)). CHO cell membranes were incubated in 50 nM Tris pH 7.4 with 0.2 nM [³H]prazosin, in a final volume of 1.02 ml for 30 minutes at 25°C, in the absence or presence of competing drugs (1 pM-10 µM). Non-specific binding was determined in the presence of 10 □M phentolamine. Incubation was stopped by addition of ice-cold Tris buffer and rapid filtration through 0.2% polyethyleneimine pretreated Schleicher & Schuell GF52 filters. Genomic clone G-21 coding for the human 5-HT_{1A}-serotoninergic receptor was stably transfected in a human cell line (HeLa) (Fargin et al., J. Biol. Chem. 284, 14848-14852 (1989)). The HeLa cells were grown as monolayers in Dulbecco's modified Eagle's medium (DMEM), supplemented with 10% foetal calf serum and gentamicin (100 □g/ml), 5% CO₂, at 37°C. The cells were detached from the growth flask at 95% confluence by a cell scraper and were lysed in ice-cold 5 mM Tris and 5-mM EDTA buffer (pH 7.4). The homogenates were centrifuged at 40000 x g x 20 minutes and the membranes were re-suspended in a small volume of ice-cold 5 mM Tris and 5-mM EDTA buffer (pH 7.4) and immediately frozen and stored at -70°C until use. On the experiment day, the cell membranes were re-suspended in a buffer containing 50 mM Tris (pH 7.4), 2.5 mM MgCl₂, 10 µM pargyline (Fargin et al., Nature 335, 358-360 (1988)). The membranes were incubated in a final volume of 1 ml for 30 minutes at 30°C with 1.2 nM [³H]8-OH-DPAT, in the absence or presence of competing test drugs; non-specific binding was determined in the presence of 10 µM 5-HT. The incubation was stopped by addition of ice-cold Tris buffer and rapid filtration through 0.2%-polyethyleneimine-pretreated Schleicher & Schuell filters.
The inhibition of specific binding of the radioligands by the drugs was analysed to estimate the IC₅₀ value by using the non-linear curve-fitting programme Allfit (De Lean et al., *Am. J. Physiol.* 235, E97-E102 (1978)). The IC₅₀ value was converted to an affinity constant (Ki) by the equation of Cheng & Prusoff (Biochem. Pharmacol. 22, 3099-3108 (1973)). Data were expressed as mean of Ki.
The compounds of the invention exhibited the desired potency and selectivity at α_{1d}-adrenoceptor, as shown in Table 1.

**Table 1.**

| *Affinity (Ki, nM) of the different compounds tested for human recombinant α*_{*1*} *adrenoceptor subtypes and 5-HT*_{*1A*} *receptor*. | | | | |
|---|---|---|---|---|
| **Example** | **Human cloned receptors** | | | |
| | **α**_{**1a**} | **α**_{**1b**} | **α**_{**1d**} | **5-HT**_{**1A**} |
| 1 | 128.30 | 9.90 | 0.13 | 36.04 |
| 2 | 77.88 | 54.08 | 3.25 | 258.10 |
| 3 | 125.55 | 193.29 | 2.91 | 69.55 |
| 4 | >1000 | 119.18 | 2.38 | 52.31 |
| 5 | 119.28 | 168.28 | 10.14 | 275.40 |
| 6 | >1000 | >1000 | 7.56 | 160.30 |
| 7 | 114.06 | 47.54 | 3.72 | 62.22 |
| 8 | 503.23 | 17.01 | 0.20 | 29.64 |
| 9 | 107.73 | 10.05 | 0.18 | 12.07 |
| 10 | 479.48 | 447.86 | 15.29 | 803.57 |
| 11 | 330.51 | 52.89 | 2.21 | 127.84 |
| 12 | 91.96 | 86.38 | 7.78 | >1000 |
| 13 | 59.82 | 43.35 | 1.23 | 104.10 |
| 14 | 68.26 | 83.61 | 3.11 | >1000 |
| 15 | 119.60 | 79.62 | 2.65 | >1000 |
| 16 | 186.68 | 13.11 | 0.41 | 206.96 |
| 17 | 118.74 | 37.57 | 1.50 | 248.19 |
| 18 | 786.36 | 915.41 | 16.38 | 432.70 |
| 19 | 53.80 | 10.62 | 0.11 | 17.43 |
| 20 | 27.21 | 15.84 | 1.43 | 91.86 |
| 24 | 705.70 | 22.75 | 0.31 | 214.00 |
| 25 | - | - | 0.66 | 40.40 |
| 26 | - | - | 0.96 | 35.33 |
| BMY-7378 | 381.05 | 68.97 | 1.43 | 0.93 |

### Example 31

### In vitro assessment of functional antagonism for α₁-adrenoceptor subtypes

### Rat aorta (α_{1D})

Thoracic aorta was obtained from Sprague Dawley rats, cut in helicoidal strips and placed in Krebs solution containing desmethylimipramine (0.1 µM), corticosterone (1 µM), propranol (1 µM) and yohimbine (0.1 µM). The strips were mounted for isotonic tension recording in an organ bath containing the above Krebs buffer enriched and aerated constantly with 95% O₂ and 5% CO₂, maintained at 37°C and loaded with 1.5 g. After an equilibration period, the strips were primed twice with 10 µM noradrenaline, then concentration/response-to-the-agonist curves were constructed before (basal) and after 30 minutes incubation with single antagonist concentrations (response). The Schild-plot parameters were evaluated by linear-regression analysis according to Tallarida and Murray (Manual of Pharmacologic Calculations with Computer Programs, 53-56, Springer-Verlag).

### Rat vas deferens (α_{1A})

Epididymal vasa deferentia were obtained from Sprague Dawley rats and placed in Krebs solution containing cocaine (10 µM) and β-oestradiol (10 µM). The vasa were mounted for isometric tension recording in an organ bath containing the above Krebs buffer enriched and aerated constantly with 95% O₂ and 5% CO₂, maintained at 37°C and loaded with 0.5 g. After an equilibration time, the tissues were primed with 10 □M noradrenaline, then a non-cumulative concentration/response-to-noradrenaline curve were constructed before (basal) and after 30 minutes incubation with single antagonist concentrations (response). The Schild-plot parameters were evaluated by linear-regression analysis according to Tallarida and Murray (Manual of Pharmacologic Calculations with Computer Programs, 53-56, Springer-Verlag).

### Guinea pig spleen (α_{1B})

Spleens were obtained from Hartley guinea pigs, cut in longitudinal strips and placed in Krebs solution containing desmethylimipramine (0.1 µM), corticosterone (1 µM), propanol (1 µM) and yohimbine (0.1 µM). The strips were mounted for isometric tension recording in an organ bath containing the above Krebs buffer enriched and aerated constantly with 95% O₂ and 5% CO₂, maintained at 37°C and loaded with 1 g. After an equilibration period, the strips were primed with 10 µM noradrenaline, then concentration/response-to-the-agonist curves were constructed before (basal) and after 30 minutes incubation with single antagonist concentrations (response). The Schild-plot parameters were evaluated by linear-regression analysis according to Tallarida and Murray (Manual of Pharmacologic Calculations with Computer Programs, 53-56, Springer-Verlag).

### Rabbit aorta pre-treated with cloroethylclonidine (α_{1L})

Aorta was obtained from White New Zealand rabbits, cut in rings and placed in Krebs solution containing desmethylimipramine (0.1 µM), corticosterone (1 µM), propanol (1 µM) and yohimbine (0.1 µM). The rings were mounted for isometric tension recording in an organ bath containing the above Krebs buffer enriched and aerated constantly with 95% O₂ and 5% CO_{2,} maintained at 37°C and loaded with 2 g. After an equilibration period, the strips were primed twice with 10 µM noradrenaline, then cloroethylclonidine (3 x 10⁻⁵ M) was added to the bath for 30 minutes followed by a 30-minute washing period (three times every 10 minutes). Cumulative concentration/response-to-noradrenaline curves were constructed before (basal) and after 30 minutes incubation with single antagonist concentrations (response). The Schild-plot parameters were evaluated by linear-regression analysis according to Tallarida and Murray (Manual of Pharmacologic Calculations with Computer Programs, 53-56, Springer-Verlag).

**Table 2.**

| *Functional affinity of α*_{*1*}*-antagonists for the different subtypes of the α*_{*1*} *adrenoceptor, expressed as pK*_{*B*} *values* | | | | |
|---|---|---|---|---|
| **Compound** | **Rat aorta α**_{**1D**} | **Rat vas deferens α**_{**1A**} | **Guinea pig spleen α**_{**1B**} | **Rabbit aorta + CEC α**_{**1L**} |
| BMY 7378 | 8.32 | 6.67^{a} | 6.55^{a} | 5.85 |
| EXAMPLE 1 | 8.99 | 5.43 | 5.55 | 5.29 |
| EXAMPLE 19 | 8.89 | 7.00 | 6.42 | 6.13 |
| a: Eltze M., *Eur. J. Pharmacol*. 260, 211-220 (1994). | | | | |

### Example 32

### Effects on cystometric parameters in conscious rats with infravesical outflow obstruction by partial urethral ligature.

### Animals

Female Sprague Dawley rats of 225-275 g body weight [Crl: CD(SD)BR, from Charles River Italia, Calco, Como] were used. The animals were housed with free access to food and water and maintained on a forced 12 hour alternating light dark cycle at 22-24°C for at least one week, except during experiment performance.

### Instruments

■ Peristaltic pump, Gilson minipuls 2.
■ Conventional pressure transducers (Marb P 82, Statham P23 ID or XL).
■ Polygraph recorder (Battaglia Rangoni KO 380 with ADC 1/T preamplifier, San-ei Rectigraph 8k or ALFOS WK-480R with BM 614 or BM 614/2 amplifier from Biomedica Mangoni).
■ Conventional isometric force transducers (Basile 7004).

### Animal preparation and surgical procedure

In order to obtain a partial obstruction of the urethra, the method previously reported by Malmgren (Malmgren A. et al., J. UroL 137, 1291-1294, 1987; Neurourol. Urodyn. 6, 371-380, 1988) was followed with minor modifications (Guarneri L. et al. Pharmacol. Res. 24, 263-272, 1991).
The rats were anaesthetised with intraperitoneal (i.p.) administration of 3 ml/kg of Equithensin solution (pentobarbital 1.215 g, chloral hydrate 5.312 g, magnesium sulphate 2.657 g, ethanol 12.5 ml, propylene glycol 49.5 ml, distilled water to 125 ml of final volume) and then the bladder and proximal urethra were exposed via an abdominal midline incision. A silk ligature was placed around the urethra and tied in the presence of an intraluminally placed indwelling polyethylene cannula with an outside diameter of 1.22 mm. After removing the polyethylene cannula, the abdominal wall was sutured and then antibiotic medication (penicillin G 200000 I.U./kg and streptomycin 260 mg/kg i.p.) was performed. After three weeks of obstruction the animals were prepared for cystometry.

### Cystometrographic preparation and recordings

The animals, anesthetized as reported above, were placed in a supine position and a midline incision was made in the shaved and cleaned abdominal wall. The urinary bladder was exposed and gently freed from adhering tissues, emptied and then cannulated, via an incision at the dome, with a polyethylene cannula (0.58 mm inside diameter and 0.96 mm outside diameter), which was permanently sutured with silk thread. For intravenous bolus injection a similar polyethylene tubing filled with physiological saline containing sodium heparin (40 I.U./ml) was inserted into the jugular vein. The cannulae were exteriorised through a subcutaneous tunnel in the retroscapular area, where they were connected with a plastic adapter, in order to avoid the risk of removal by the animal. Two days after bladder catheter implantation, the animals, fasted overnight, were placed in Bollman's cages or in particular plastic cages which allowed some lateral and back and forth movement of the rats, allowing measurements of micturition volume (MV). After a stabilisation period of 20 minutes, the free tip of the bladder cannula was connected to a pressure transducer and to a peristaltic pump for a continuous infusion of warm saline solution (37° C), into the urinary bladder. In order to shorten the time-period of saline infusion necessary to provoke micturition in the obstructed rats, a filling rate of the bladder of 10 ml/h was chosen. During infusion of saline into the bladder, the intravesical pressure signal and urine volume micturated were continuously recorded on chart polygraph. The effect of the compounds on unstable bladder was evaluated from the cystometrograms of the obstructed rats, where the number and the mean amplitude of the spontaneous bladder contractions present during bladder filling without urine emission and termed "non-voiding contractions", were evaluated in the 2 minute time before micturition. In addition, the following urodynamic parameters were investigated: bladder volume capacity (BVC), micturition pressure (MP) and micturition volume (MV). BVC (in ml) was defined as the volume of saline infused into the bladder from the start of the infusion to micturition. MP (in mmHg) was defined as the maximal intravesical pressure induced by the contraction of the detrusor during micturition. MV (in ml), defined as the volume of expelled urine during each single micturition, was recorded by means of a force displacement transducer connected to the polygraph and measuring the urine collected in a small reservoir placed under the cage. Basal values of the parameters above reported were evaluated as the mean of the values obtained in two complete and reproducible cystometrograms before treatment (time 0). Then the animals were treated intravenously by bolus injection with the test compound under continuous infusion of saline into the bladder.
The parameter changes after compound injection (first cystometrogram and those recorded approximately after 30 and 60 minutes) were compared with the values recorded before injection (basal values).

### Results

Cystometry in conscious rats with partial bladder-outlet obstruction revealed detrusor contractions during filling which are ineffective in urine expulsion (non-voiding contractions). As reported in the following table, a 0.3 mg/kg i.v. dose of the compound of Example 1 produced rapid decrease in the frequency and amplitude of the non-voiding contractions immediately after the injections. The duration of the effects was about 30 minutes on the frequency and at least 60 minutes on the amplitude. With regard to the other urodynamic parameters investigated, BVC mean values were changed by 20-30%; MV changes followed the same modifications whereas no significant changes were observed in MP values.
We may thus say that the tested product is capable of reducing contractility of the unstable bladder.

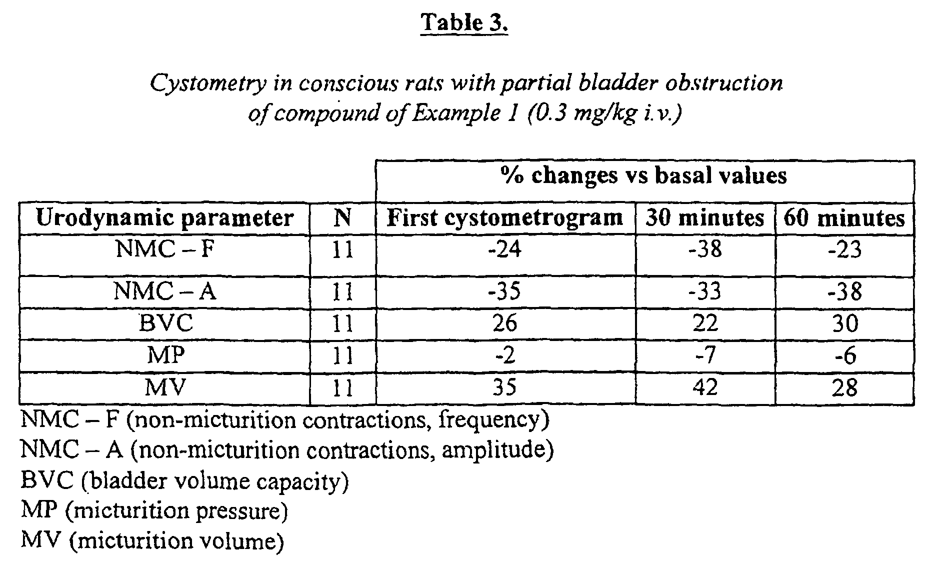

### Effective amounts

The following represent guidelines to effective parenteral or intravenous dose ranges expressed in mg/kg of body weight per day for the compounds of the invention:

| | |
|---|---|
| General | 0.01-20 |
| Preferred | 0.02-10 |
| Most Preferred | 1-4 |

Most preferred values refer to oral dosing. Intravenous dosages should be 10 to 100 fold lower.
Selective use dosages, i.e. dosages that are active in the lower urinary tract without a substantial effect on the blood pressure, depend on the particular compound employed. Generally, in the case of a compound selective in increasing bladder volume capacity, up to four times the amount of the effective dose used can be administered without substantial effect on blood pressure. Further refinements and optimisation of dosages are possible using no more than routine experiments. The active compounds of the invention may be orally administered, for example, with an inert diluent or with an edible carrier, or they may be enclosed in gelatine capsules, or they may be compressed into tablets. For the purpose of oral therapeutic administration, the active compounds of the invention may be incorporated with excipients and used in the form of tablets, troches, capsules, elixirs, suspensions, syrups, wafers, chewing gum and the like. These preparations should contain at least 0.5% of active compounds, but the amount of active ingredient may be varied depending upon the particular form and may conveniently be between 5% and about 70% of the weight of the unit. The amount of active compound in such compositions is such that a suitable dosage will be obtained although the desired dosage can be obtained by administering a plurality of dosage forms. Preferred compositions and preparations according to the invention are prepared so that an oral dosage unit form contains between 1.0-300 milligrams of active compound. The tablets, pills, capsules, troches and the like may also contain, for example, the following ingredients: a binder such as microcrystalline cellulose, gum tragacanth or gelatine; an excipient such as starch or lactose, a disintegrating agent such as alginic acid, sodium starch glycolate, cornstarch and the like; a lubricant such as magnesium stearate or hydrogenated castor oil, a glidant such as colloidal silicon dioxide; and a sweetening agent such as sucrose or saccharin may be added or a flavouring agent such as peppermint, methyl salicylate or orange flavouring. When the dosage unit form is a capsule, it may contain, in addition to materials of the above type, a liquid carrier such as a fatty oil. Other dosage unit forms may contain other various materials which modify the physical form of the dosage unit, for example, as coatings. Thus, tablets or pills may be coated with sugar, shellac or other enteric-coating agents. A syrup may contain, in addition to the active compounds, sucrose as a sweetening agent and certain preservatives, dyes, colouring and flavours. The materials used in preparing these various compositions should be pharmaceutically pure and non-toxic in the amounts used. For the purpose of parenteral therapeutic administration, the active compounds of the invention may be incorporated into a solution or suspension. These preparations should contain at least 0.1% of active compound, but may be varied between 0.5 and about 30% of the weight thereof. The amount of active compound in such compositions is such that a suitable dosage will be obtained. Preferred compositions and preparations according to the present inventions are prepared so that a parenteral dosage unit contains between 0.2 and 100 milligrams of active compound. The solutions or suspensions may also include the following components: a sterile diluent such as water for injection, saline solution, polyethylene glycols, glycerine, propylene glycol or other synthetic solvents; antibacterial agents such as benzyl alcohol or the parabens; antioxidants such as ascorbic acid or sodium bisulphite; chelating agents such as tetraacetic acid; buffers such as acetates; citrates or phosphates; and agents for the adjustment of tonicity such as sodium chloride or dextrose. The parenteral multiple-dose vials may be of glass or plastics material. Additional compositions suitable for administration by various routes and containing compounds according to the present invention are also within the scope of the invention. Dosage forms, additional ingredients and routes of administration contemplated herein include those disclosed in U.S. Patents Nos. 4,089,969 and 5,091,182, all incorporated by reference in their entirety.

## Claims

1. A compound having the general formula I 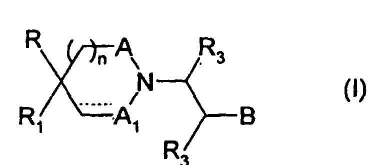 wherein
each of R and R₁ independently represents a hydrogen atom or a lower alkyl group having from 1 to 4 carbon atoms, or R and R₁ together form an alkylene bridge of the formula (CH₂)₂₋₆,
n is an integer from 0 to 1,
...... represents a single or a double bond,
A represents a carbonyl group or a methylene group,
A₁ represents a carbonyl group or a methylene group or a methyne group,
each R₃ independently represents a hydrogen atom or a lower alkyl group having from 1 to 4 carbon atoms, and
B represents one of the following groups 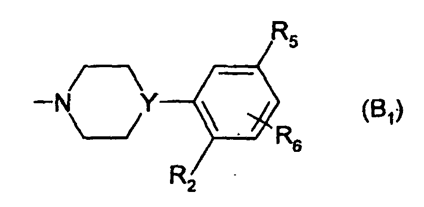 wherein
Y represents a nitrogen atom or a methyne group,
R₂ represents a halogen or a lower alkyl group having from 1 to 4 carbon atoms or a cyano group,
R₅ represents a halogen atom or a lower alkyl group having from 1 to 4 carbon atoms or a polyfluoroalkyl group or a nitro group, and
R₆ represents a hydrogen or halogen atom; or 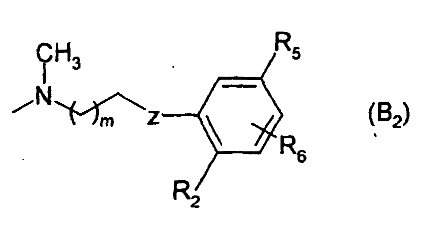 wherein
m is an integer from I to 3,
Z represents an oxygen or sulphur atom or an amino or methylamino group, and
R₂, R₅ and R₆ have the same meanings as above;
with the first proviso that if R and R₁ together form an alkylene bridge, A and A₁ both represent carbonyl groups, n is 1, the heterocyclic ring is saturated and Y represents a nitrogen atom, then not more than one of R₂, R₅ and R₆ represents a fluorine atom,
and with the second proviso that the compound is not 8-{2-[4-(5-chloro-2-methylphenyl)-1-piperazinyl]-ethyl}-8-azaspiro[4.5]decane-7,9-dione;
or an enantiomer, diastereoisomer, N-oxide or pharmaceutically acceptable salt of such a compound.

2. A compound according to claim 1 in which R and R₁ together represent a trimethylene, tetramethylene, pentamethylene or hexamethylene group.

3. A compound according to claim 1 or claim 2 in which A and A₁ both represent carbonyl groups, or in which one of A and A₁ represents a carbonyl group and the other of A and A₁ represents a methylene group.

4. A compound according to any preceding claim in which B represents a B₁ group, Y represents a nitrogen atom and R₂ and R₅ both represent chlorine atoms.

5. Any one of the following compounds:
8-{2-[4-(2,5-Dichlorophenyl)-1-piperazinyl]-ethyl}-8-azaspiro[4.5]decane-7,9-dione,
1-{2-[4-(2,5-Dichlorophenyl)-1-piperazinyl]-ethyl}-piperidinyl-2,6-dione,
8-{2-[4-(2-Chloro-5-methylphenyl)-1-piperazinyl]-ethyl}-8-azaspiro[4.5]-decane-7,9-dione,
8-{2-[4-(2-Fluoro-5-methylphenyl)-1-piperazinyl]-ethyl}-8-azaspiro[4.5]-decane-7,9-dione,
8-{2-[4-(2,5-Dimethylphenyl)-1-piperazinyl]-ethyl}-8-azaspiro[4.5]decane-7,9-dione,
8-{2-[4-(2-Fluoro-5-trifluoromethylphenyl)-1-piperazinyl]-ethyl}-8-azaspiro[4.5]decane-7,9-dione,
8- {2-[4-(2,5-Dibromophenyl)-1-piperazinyl]-ethyl}-8-azaspiro[4.5]decane-7,9-dione,
8-{2-[4-(5-Chloro-2-cyanophenyl)-1-piperazinyl]-ethyl}-8-azaspiro[4.5]decane-7,9-dione,
8-{2-[4-(2-Chloro-5-fluorophenyl)-1-piperazinyl]-ethyl}-8-azaspiro[4.5]decane-7,9-dione,
8-{2-[4-(2-Chloro-5-iodophenyl)-1-piperazinyl]-ethyl}-8-azaspiro[4.5]decane-7,9-dione,
3-{2-[4-(2,5-Dichlorophenyl)-1-piperazinyl]-ethyl}-3-azaspiro[5.5]undecane-2,4-dione,
1-{2-[4-(2,5-Dichlorophenyl)-1-piperazinyl]-ethyl}-pyrrolidinyl-2,5-dione,
1-{2-[4-(2,5-Dichlorophenyl)-1-piperazinyl]-ethyl}-4-ethyl-4-methylpiperidinyl-2,6-dione,
2-{2-[4-(2,5-Dichlorophenyl)-1-piperazinyl]-ethyl}-2-azapiro[4.4]nonane-1,3-dione,
7-{2-[4-(2,5-Dichlorophenyl)-1-piperazinyl]-ethyl}-7-azaspiro[3.5]nonane-6,8-dione,
8- {2-[4-(2,5-Dichlorophenyl)-1-piperazinyl]-1-methylethyl}-8-azaspiro[4.5]decane-7,9-dione,
8- {2-[4-(2,5-Dichlorophenyl)-1-piperidinyl]-ethyl}-8-azaspiro[4.5]decane-7,9-dione,
N-(2,5-Dichlorophenyl)-N'-{2-(7,9-dioxo-8-azaspiro[4.5]decane-8-yl)-ethyl}-N,N'-dimethyl-1,2-diaminoethane,
8-{2-[4-(2,5-Dichlorophenyl)-1-piperazinyl]-ethyl}-8-azaspiro[4.5]decane-7-one,
8-{2-[4-(2,5-Dichlorophenyl)-1-piperazinyl]-ethyl}-8-azaspiro[4.5]dec-9,10-en-7-one,
8-{2-[4-(2,5-Dichlorophenyl)-1-piperazinyl]-ethyl}-8-azaspiro[4.5]decane,
8-{2-[4-(2-Chloro-5-nitrophenyl)-1-piperazinyl]-ethyl}-8-azaspiro[4.5]decane-7,9-dione,
8-{2-[4-(2,5-Dichlorophenyl)-1-piperazinyl]-ethyl}-8-azaspiro[4.5]decane-7-one N¹-oxide,
8-{2-[4-(2,4,5-Trifluorophenyl)-1-piperazinyl]-ethyl}-8-azaspiro[4.5]decane-7-one,
3-{2-[4-(2,5-Dichlorophenyl)-1-piperazinyl]-ethyl}-3-azaspiro[5.5]undecane-2-one,
3-{2-[4-(2,5-Dichlorophenyl)-1-piperazinyl]-ethyl}-3-azaspiro[5.6]dodecane-2,4-dione,
8-{2-[4-(5-Chloro-2-cyanophenyl)-1-piperazinyl]-ethyl}-8-azaspiro[4.5]decane-7-one,
8-{2-[4-(5-Chloro-2-methylphenyl)-1-piperazinyl]-ethyl}-8-azaspiro[4.5]decane-7-one and
8-{2-[4-(5-Chloro-2-fluorophenyl)-1-piperazinyl]-ethyl}-8-azaspiro[4.5]decane-7-one.

6. A pharmaceutical composition comprising a compound according to any preceding claim, or an enantiomer, diastereoisomer, N-oxide or pharmaceutically acceptable salt of such a compound, in admixture with a pharmaceutically acceptable diluent or carrier.

7. A method for the preparation of a compound having the general formula I as defined in claim 1, the method comprising alkylating an amine BH, in which B is as defined in claim 1, with a haloalkyl intermediate of the general formula II 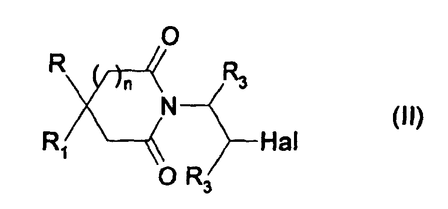 where R, R₁, R₃ and n are as defined in claim 1 and Hal represents a halogen atom.

8. A method according to claim 7 in which the reaction is carried out without solvent in the presence of a base at a temperature ranging from 100 to 180°C.

9. A method for the preparation of a compound having the general formula I as defined in claim 1, the method comprising acylating an amine BCH₂CH₂NH₂, in which B is as defined in claim 1, with an anhydride of the general formula 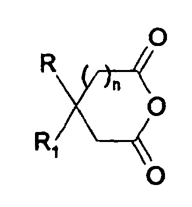 where R, R₁ and n are as defined in claim 1.

10. A method according to claim 9 in which the reaction is carried out in an apolar aprotic solvent in the presence of a promoting agent at a temperature ranging from 80°C to the solvent's reflux temperature.

11. A method for the preparation of a compound having the general formula I in which A and A₁ both represent carbonyl groups, B represents the group B₁, R₃ represents a hydrogen atom and R, R₁, n and ...... are as defined in claim 1, the method comprising amidifying a diacid of the general formula XXI 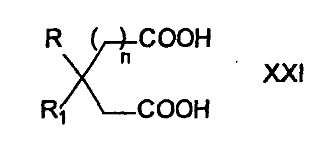 in which R, R₁ and n are as defined in claim 1, with an amine BCH₂CH₂NH₂ in which B is as defined in claim 1, and dehydrating the resulting amide intermediate.

12. A method according to claim 11 in which the amidification is carried out in a polar aprotic solvent in the presence of a condensing agent at a temperature ranging from 10 to 40°C.

13. A method according to claim 11 and claim 12 in which the dehydration is effected using an anhydride without solvent, at a temperature ranging from 80 to 120°C.

14. A method for the preparation of a compound having the general formula I in which A represents a methylene group, B represents the group B₁ or B₂, R₃ represents a hydrogen atom and A₁, R, R₁, n and ...... are as defined in claim 1, the method comprising reacting an ester aldehyde of the general formula XXII 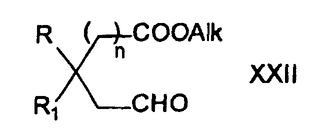 in which R, R₁ and n are as defined in claim 1, with an amine BCH₂CH₂NH₂ in which B is as defined in claim 1.

15. A method according to claim 14 in which the reaction is carried out under reductive conditions using a metal borohydride in a polar protic solvent at a temperature ranging from -5 to 25°C, to give a compound of formula I in which ...... represents a single bond and A₁ represents a methylene group.

16. A method according to claim 14 in which the reaction is carried out in an apolar aprotic solvent in the presence of an acid catalyst at the solvent's reflux temperature, to give a compound of formula I in which ...... represents a double bond and A₁ represents a methyne group.

17. A method for the preparation of a compound having the general formula I as defined in claim 1, the method comprising condensing a diester of the general formula 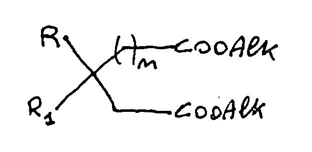 where R, R₁ and n are as defined in claim 1 with an amine BCH₂CH₂NH₂ in which B is as defined in claim 1.

18. A method according to claim 17 in which the condensation is effected without solvent at a temperature ranging from 80 to 160°C.

19. A method for the preparation of a compound having the general formula I in which A and A₁ both represent methylene groups, ...... represents a single bond and B, R, R₁, R₃ and n are as defined in claim 1, the method comprising reducing a compound having the general formula I in which A and A₁ both represent carbonyl groups and B, R, R₁, R₃, n and ...... are as defined in claim 1.

20. A method according to claim 19 in which the reduction is effected using a metal borohydride in the presence of a Lewis catalyst in a polar aprotic solvent at a temperature ranging from -5 to 25°C.

## Patentansprüche

1. Verbindung der allgemeinen Formel I 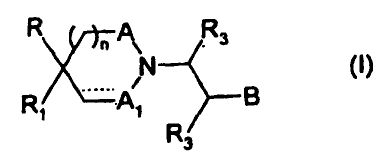 worin
jedes R und R₁ unabhängig ein Wasserstoffatom oder eine niedere Alkylgruppe mit 1 bis 4 Kohlenstoffatomen darstellt, oder R und R₁ zusammen eine Alkylenbrücke der Formel (CH₂)₂₋₆ bilden,
n eine ganze Zahl von 0 bis 1 ist,
...... eine Einfach- oder eine Doppelbindung darstellt,
A eine Carbonylgruppe oder eine Methylengruppe darstellt,
A₁ eine Carbonylgruppe oder eine Methylengruppe oder eine Methingruppe darstellt,
jedes R₃ unabhängig ein Wasserstoffatom oder eine niedere Alkylgruppe mit 1 bis 4 Kohlenstoffatomen darstellt und
B eine der nachfolgenden Gruppen darstellt 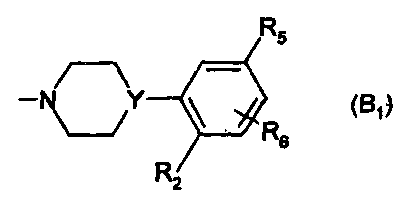 worin
Y ein Stickstoffatom oder eine Methingruppe darstellt,
R₂ ein Halogen oder eine niedere Alkylgruppe mit 1 bis 4 Kohlenstoffatomen oder eine Cyanogruppe darstellt,
R₅ ein Halogenatom oder eine niedere Alkylgruppe mit 1 bis 4 Kohlenstoffatomen oder ein Polyfluoralkylgruppe oder eine Nitrogruppe darstellt und
R₆ ein Wasserstoff- oder Halogenatom darstellt, oder 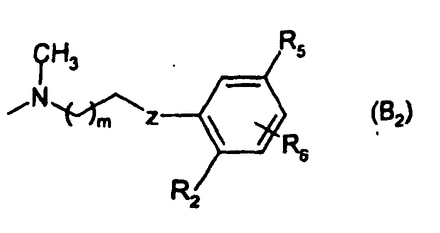 worin
m eine ganze Zahl von 1 bis 3 ist,
Z ein Sauerstoff- oder Schwefelatom oder eine Amino- oder Methylaminogruppe darstellt und
R₂, R₅ und R₆ dieselben Bedeutungen wie oben haben;
mit der ersten Bedingung, dass wenn R und R₁ zusammen eine Alkylenbrücke bilden, A und A₁ beide Carbonylgruppen darstellen, n 1 ist, der heterozyklische Ring gesättigt ist und Y ein Stickstoffatom darstellt, dann nicht mehr als einer von R₂, R₅ und R₆ ein Fluoratom darstellt,
und mit der zweiten Bedingung, dass die Verbindung nicht 8-{-2-[4-(5-Chlor-2-methylphenyl)-1-piperazinyl]-ethyl}-8-azaspiro[4.5]decan-7,9-dion ist, oder ein Enantiomer, Diastereoisomer, N-Oxid oder pharmazeutisch verträgliches Salz einer solchen Verbindung ist.

2. Verbindung nach Anspruch 1, in der R und R₁ zusammen eine Trimethylen-, Tetramethylen-, Pentamethylen- oder Hexamethylengruppe darstellen.

3. Verbindung nach Anspruch 1 oder Anspruch 2, in der A und A₁ beide Carbonylgruppen darstellen oder in der einer von A und A₁ eine Carbonylgruppe darstellt und der andere von A und A₁ eine Methylengruppe darstellt.

4. Verbindung nach jedem vorangehenden Anspruch, in der B eine B₁-Gruppe darstellt, Y ein Stickstoffatom darstellt und R₂ und R₅ beide Chloratome darstellen.

5. Jede der nachfolgenden Verbindungen:
8-{2-[4-(2,5-Dichlorphenyl)-1-piperazinyl]-ethyl}-8-azaspiro[4.5]decan-7,9 dion,
1-{2-[4-(2,5-Dichlorphenyl)-1-piperazinyl]-ethyl}-piperidinyl-2,6-dion,
8-{2-[4-(2-Chlor-5-methylphenyl)-1-piperazinyl]-ethyl}-8-azaspiro [4.5]-decan-7,9-dion,
8-{2-[4-(2-Fluor-5-methylphenyl)-1-piperazinyl]-ethyl}-8-azaspiro[4.5]-decan-7,9-dion,
8-{2-[4-(2,5-Dimethylphenyl)-1-piperazinyl]-ethyl}-8-azaspiro[4.5]decan-7,9-dion,
8-{2-[4-(2-Fluor-5-trifluormethylphenyl)-1-piperazinyl]-ethyl}-8-azaspiro [4.5] decan-7,9-dion,
8-{2-[4-(2,5-Dibromphenyl)-1-piperazinyl]-ethyl}-8-azaspiro[4.5]decan-7,9-dion,
8-{2-[4-(5-Chlor-2-cyanophenyl)-1-piperazinyl]-ethyl}-8-azaspiro[4.5]decan-7,9-dion,
8-{2-[4-(2-Chlor-5-fluorphenyl)-1-piperazinyl]-ethyl}-8-azaspiro[4.5]decan-7,9-dion,
8-{2-[4-(2-Chlor-5-jodphenyl)-1-piperazinyl]-ethyl}-8-azaspiro[4.5]decan-7,9-dion,
3-{2-[4-(2,5-Dichlorphenyl)-1-piperazinyl]-ethyl}-3-azaspiro [5.5] undecan-2,4-dion,
1-{2-[4-(2,5-Dichlorphenyl)-1-piperazinyl]-ethyl}-pyrrolidinyl-2,5-dion,
1-{2-[4-(2,5-Dichlorphenyl)-1-piperazinyl]-ethyl}-4-ethyl-4-methylpiperidinyl-2,6-dion,
2-{2-[4-(2,5-Dichlorphenyl)-1-piperazinyl]-ethyl}-2-azaspiro[4.4]nonan-1,3-dion,
7-{2-[4-(2,5-Dichlorphenyl)-1-piperazinyl]-ethyl}-7-azaspiro[3.5]nonan-6,8-dion,
8-{2-[4-(2,5-Dichlorphenyl)-1-piperazinyl]-1-methylethyl}-8-azaspiro[4.5]decan-7,9-dion,
8-{2-[4-(2,5-Dichlorphenyl)-1-piperazinyl]-ethyl}-8-azaspiro[4.5]decan-7,9-dion,
N-(2,5-Dichlorphenyl)-N'-{2-(7,9-dioxo-8-azaspiro[4.5]decan-8-yl)-ethyl}-N,N'-dimethyl-1,2-diaminoethan,
8-{2-[4-(2,5-Dichlorphenyl)-1-piperazinyl]-ethyl}-8-azaspiro[4.5]decan-7-on,
8-{2-[4-(2,5-Dichlorphenyl)-1-piperazinyl]-ethyl}-8-azaspiro[4.5]dec-9,10-en-7-on,
8-{2-[4-(2,5-Dichlorphenyl)-1-piperazinyl]-ethyl}-8-azaspiro[4.5]decan,
8-{2-[4-(2-Chlor-5-nitrophenyl)-1-piperazinyl]-ethyl}-8-azaspiro[4.5]decan-7,9-dion,
8-{2-[4-(2,5-Dichlorphenyl)-1-piperazinyl]-ethyl}-8-azaspiro[4.5]decan-7-on-N¹oxid,
8-{2-[4-(2,4,5-Trifluorphenyl)-1-piperazinyl]-ethyl}-8-azaspiro[4.5]decan-7-on,
3-{2-[4-(2,5-Dichlorphenyl)-1-piperazinyl]-ethyl}-3-azaspiro[5.5]undecan-2-on,
3-{2-[4-(2,5-Dichlorphenyl)-1-piperazinyl]-ethyl}-3-azaspiro[5.6]dodecan-2,4 dion,
8-{2-[4-(5-Chlor-2-cyanophenyl)-1-piperazinyl]-ethyl}-8-azaspiro[4.5]decan-7-on,
8-{2-[4-(5-Chlor-2-methylphenyl)-1-piperazinyl]-ethyl}-8-azaspiro[4.5]decan-7-on,
und
8-{2-[4-(5-Chlor-2-fluorphenyl)-1-piperazinyl]-ethyl}-8-azaspiro[4.5]decan-7-on.

6. Pharmazeutische Zusammensetzung, umfassend eine Verbindung gemäß jedem vorangehenden Anspruch, oder ein Enantiomer, Diastereoisomer, N-Oxid oder pharmazeutisch verträgliches Salz einer solchen Verbindung, in Beimischung mit einem pharmazeutisch verträglichen Verdünnungsmittel oder Träger.

7. Verfahren zur Herstellung einer Verbindung der allgemeinen Formel I wie in Anspruch 1 definiert, wobei das Verfahren umfasst, dass man ein Amin BH, in dem B wie in Anspruch 1 definiert ist, mit einem Halogenalkyl-Zwischenprodukt der allgemeinen Formel II alkyliert 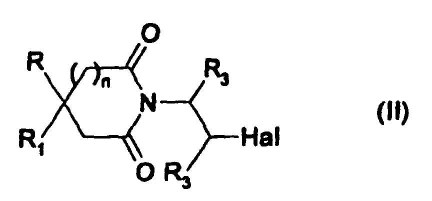 worin R, R₁ R₃ und n wie in Anspruch 1 definiert sind und Hal ein Halogenatom darstellt.

8. Verfahren nach Anspruch 7, worin die Reaktion ohne Lösungsmittel in Gegenwart einer Base bei einer Temperatur im Bereich von 100 bis 180°C durchgeführt wird.

9. Verfahren zur Herstellung einer Verbindung der allgemeinen Formel I wie in Anspruch 1 definiert, wobei das Verfahren umfasst, dass man ein Amin BCH₂CH₂NH₂, worin B wie in Anspruch 1 definiert ist, mit einem Anhydrid der allgemeinen Formel 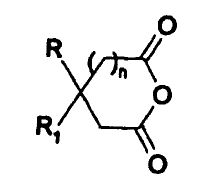 acyliert, worin R, R₁ und n wie in Anspruch 1 definiert sind.

10. Verfahren nach Anspruch 9, worin die Reaktion in einem apolaren aprotischen Lösungsmittel in Gegenwart eines Reaktionsbeförderungsmittels bei einer Temperatur im Bereich von 80°C bis zu der Rückflusstemperatur des Lösungsmittels durchgeführt wird.

11. Verfahren zur Herstellung einer Verbindung der allgemeinen Formel I, in der A und A₁ beide Carbonylgruppen darstellen, B die Gruppe die B₁ darstellt, R₃ ein Wasserstoffatom darstellt und R, R₁, n und ...... wie in Anspruch 1 definiert sind, wobei das Verfahren die Amidifizierung einer Disäure der allgemeinen Formel XXI 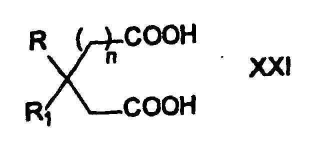 in der R, R₁ und n wie in Anspruch 1 definiert sind, mit einem Amin BCH₂CH₂NH₂ umfasst, in dem B wie in Anspruch 1 definiert ist, und man das resultierende Amidzwischenprodukt dehydratisiert.

12. Verfahren nach Anspruch 11, in dem die Amidifizierung in einem polaren aprotischen Lösungsmittel in Gegenwart eines Kondensationsmittels bei einer Temperatur im Bereich von 10 bis 40°C durchgeführt wird.

13. Verfahren nach Anspruch 11 oder Anspruch 12, in dem die Dehydratisierung durch Verwendung eines Anhydrids ohne Lösungsmittel bei einer Temperatur im Bereich von 80 bis 120°C bewirkt wird.

14. Verfahren zur Herstellung einer Verbindung der allgemeinen Formel I, in der A eine Methylengruppe darstellt, B die Gruppe B₁ oder B₂ darstellt, R₃ ein Wasserstoffatom darstellt und A₁, R, R₁, n und ...... wie in Anspruch 1 definiert sind, wobei das Verfahren umfasst, dass man ein Esteraldehyd der allgemeinen Formel XXII 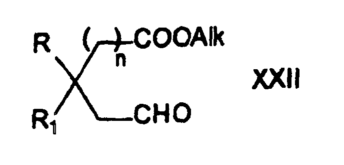 in der R, R₁ und n wie in Anspruch 1 definiert sind, mit einem Amin BCH₂CH₂NH₂ umsetzt, worin B wie in Anspruch 1 definiert ist.

15. Verfahren nach Anspruch 14, in dem die Reaktion unter reduktiven Bedingungen unter Verwendung eines Metallborhydrids in einem polaren protischen Lösungsmittel bei einer Temperatur im Bereich von -5 bis 25°C durchgeführt wird, um eine Verbindung der Formel I zu ergeben, in der ...... eine Einfachbindung darstellt und A₁ eine Methylengruppe darstellt.

16. Verfahren nach Anspruch 14, in dem die Reaktion in einem apolaren aprotischen Lösungsmittel in Gegenwart eines Säurekatalysators bei der Rückflusstemperatur des Lösungsmittels durchgeführt wird, um eine Verbindung der Formel I zu ergeben, in der ...... eine Doppelbindung darstellt und A₁ eine Methingruppe darstellt.

17. Verfahren zur Herstellung einer Verbindung der allgemeinen Formel I wie in Anspruch 1 definiert, wobei das Verfahren umfasst, dass man einen Diester der allgemeinen Formel 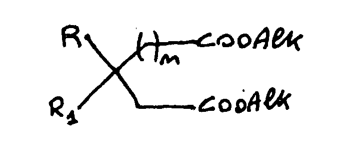 worin R, R₁ und n wie in Anspruch 1 definiert sind, mit einem Amin BCH₂CH₂NH₂ umsetzt, in dem B wie in Anspruch 1 definiert ist.

18. Verfahren nach Anspruch 17, in dem die Kondensation ohne Lösungsmittel bei einer Temperatur im Bereich von 80 bis 160°C bewirkt wird.

19. Verfahren zur Herstellung einer Verbindung der allgemeinen Formel I, in der A und A₁ beide Methylengruppen darstellen, ...... eine Einfachbindung darstellt und B, R, R₁, R₃ und n wie in Anspruch 1 definiert sind, wobei das Verfahren umfasst, dass man eine Verbindung der allgemeinen Formel I, in der A und A₁ beide Carbonylgruppen darstellen und B, R, R₁, R₃, n und ...... wie in Anspruch 1 definiert sind, reduziert.

20. Verfahren nach Anspruch 19, in dem die Reduktion durch Verwendung eines Metallborhydrids in Gegenwart eines Lewis-Katalysators in einem polaren aprotischen Lösungsmittel bei einer Temperatur im Bereich von -5 bis 25°C bewirkt wird.

## Revendications

1. Composé ayant la formule générale I 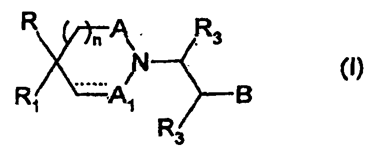 dans laquelle
chacun parmi R et R₁ représente indépendamment un atome d'hydrogène ou un groupe alkyle inférieur ayant de 1 à 4 atomes de carbone, ou R et R₁ forment ensemble un pont alkylène de formule (CH₂)₂₋₆,
n est un entier de 0 à 1,
...... représente une liaison simple ou double,
A représente un groupe carbonyle ou un groupe méthylène,
A₁ représente un groupe carbonyle ou un groupe méthylène ou un groupe méthyne,
chaque R₃ représente indépendamment un atome d'hydrogène ou un groupe alkyle inférieur ayant de 1 à 4 atomes de carbone, et
B représente l'un des groupes suivants 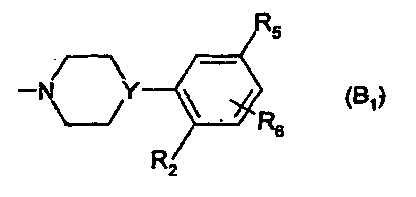 où
Y représente un atome d'azote ou un groupe méthyne,
R₂ représente un halogène ou un groupe alkyle inférieur ayant de 1 à 4 atomes de carbone ou un groupe cyano,
R₅ représente un atome d'halogène ou un groupe alkyle inférieur ayant de 1 à 4 atomes de carbone ou un groupe polyfluoroalkyle ou un groupe nitro, et
R₆ représente un atome d'hydrogène ou d'halogène ; ou 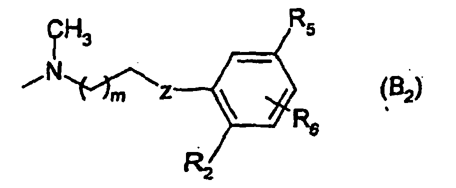 où
m est un entier de 1 à 3,
Z représente un atome d'oxygène ou de soufre ou un groupe amino ou méthylamino, et
R₂, R₅ et R₆ ont les mêmes significations que ci-dessus ;
avec pour première condition que si R et R₁ forment ensemble un pont alkylène, A et A₁ représentent tous les deux des groupes carbonyles, n est 1, le noyau hétérocyclique est saturé et Y représente un atome d'azote, alors pas plus d'un parmi R₂, R₅ et R₆ ne représente un atome de fluor,
et avec pour seconde condition que le composé n'est pas la 8-{2-[4-(5-chloro-2-méthylphényl)-1-pipérazinyl]éthyl}-8-azaspiro[4.5]décane-7,9-dione ;
ou un énantiomère, diastéréoisomère, N-oxyde ou sel pharmaceutiquement acceptable d'un tel composé.

2. Composé selon la revendication 1, dans lequel R et R₁ représentent ensemble un groupe triméthylène, tétra-méthylène, pentaméthylène ou hexaméthylène.

3. Composé selon la revendication 1 ou la revendication 2, dans lequel A et A₁ représentent tous les deux des groupes carbonyles, ou dans lequel l'un parmi A et A₁ représente un groupe carbonyle et l'autre parmi A et A₁ représente un groupe méthylène.

4. Composé selon l'une quelconque des revendications précédentes, dans lequel B représente un groupe B₁, Y représente un atome d'azote, et R₂ et R₅ représentent tous les deux des atomes de chlore.

5. L'un quelconque des composés suivants :
8-{2-[4-(2,5-Dichlorophényl)-1-pipérazinyl]-éthyl}-8-azaspiro[4.5]décane-7,9-dione,
1-{2-[4-(2,5-Dichlorophényl)-1-pipérazinyl]-éthyl}-pipéridinyl-2,6-dione,
8-{2-[4-(2-Chloro-5-méthylphényl)-1-pipérazinyl]-éthyl}-8-azaspiro[4.5]-décane-7,9-dione,
8-{2-[4-(2-Fluoro-5-méthylphényl)-1-pipérazinyl]-éthyl}-8-azaspiro[4.5]-décane-7,9-dione,
8-{2-[4-(2,5-Diméthylphényl)-1-pipérazinyl]-éthyl}-8-azaspiro[4.5]décane-7,9-dione,
8-{2-[4-(2-Fluoro-5-trifluorométhylphényl)-1-pipérazinyl]-éthyl}-8-azaspiro[4.5]décane-7,9-dione,
8-{2-[4-(2,5-Dibromophényl)-1-pipérazinyl]-éthyl}-8-azaspiro[4.5]décane-7,9-dione,
8-{2-[4-(5-Chloro-2-cyanophényl)-1-pipérazinyl]-éthyl}-8-azaspiro[4.5]décane-7,9-dione,
8- {2-[4-(2-Chloro-5-fluorophényl)-1-pipérazinyl]-éthyl}-8-azaspiro[4.5]décane-7,9-dione,
8-{2-[4-(2-Chloro-5-iodophényl)-1-pipérazinyl]-éthyl}-8-azaspiro[4.5]décane-7,9-dione,
3-{2-[4-(2,5-Dichlorophényl)-1-pipérazinyl]-éthyl}-3-azaspiro[5.5]andécane-2,4-dione,
1-{2-[4-(2,5-Dichlorophényl)-1-pipérazinyl]-éthyl}-pyrrolidinyl-2,5-dione,
1-{2-[4-(2,5-Dichlorophényl)-1-pipérazinyl]-éthyl}-4-éthyl-4-méthylpipéridinyl-2,6-dione,
2-{2-[4-(2,5-Dichlorophényl)-1-pipérazinyl]-éthyl}-2-azaspiro[4.4]nonane-1,3-dione,
7-{2-[4-(2,5-Dichlorophényl)-1-pipérazinyl]-éthyl}-7-azaspiro[3.5]nonane-6,8-dione,
8- {2-[4-(2,5-Dichlorophényl)-1-pipérazinyl]-1-méthyléthyl}-8-azaspiro[4.5]décane-7,9-dione,
8- {2-[4-(2,5-Dichlorophényl)-1-pipéridinyl]-éthyl}-8-azaspiro[4.5]décane-7,9-dione,
N-(2,5-Dichlorophényl)-N'-{2-(7,9-dioxo-8-azaspiro[4.5]décane-8-yl)-éthyl}-N,N'-diméthyl-1,2-diaminoéthane,
8-{2-[4-(2,5-Dichlorophényl)-1-pipérazinyl]-éthyl}-8-azaspiro[4.5]décane-7-one,
8-{2-[4-(2,5-Dichlorophényl)-1-pipérazinyl]-éthyl}-8-azaspiro[4.5]déc-9,10-én-7-one,
8-{2-[4-(2,5-Dichlorophényl)-1-pipérazinyl]-éthyl}-8-azaspiro[4.5]décane,
8-{2-[4-(2-Chloro-5-nitrophényl)-1-pipérazinyl]-éthyl}-8-azaspiro[4.5]décane-7,9-dione,
8-{2-[4-(2,5-Dichlorophényl)-1-pipérazinyl]-éthyl}-8-azaspiro[4.5]décane-7-one N¹-oxide,
8-{2-[4-(2,4,5-Trifluorophényl)-1-pipérazinyl]-éthyl}-8-azaspiro[4.5]décane-7-one,
3-{2-[4-(2,5-Dichlorophényl)-1-pipérazinyl]-éthyl}-3-azaspiro[5.5]undécane-2-one,
3-{2-[4-(2,5-Dichlorophényl)-1-pipérazinyl]-éthyl}-3-azaspiro[5.6]dodécane-2,4-dione,
8-{2-[4-(5-Chloro-2-cyanophényl)-1-pipérazinyl]-éthyl}-8-azaspiro[4.5]décane-7-one,
8-{2-[4-(5-Chloro-2-méthylphényl)-1-pipérazinyl]-éthyl}-8-azaspiro[4.5]décane-7-one et
8-{2-[4-(5-Chloro-2-fluorophényl)-1-pipérazinyl]-éthyl}-8-azaspiro[4.5]décane-7-one.

6. Composition pharmaceutique comprenant un composé selon l'une quelconque des revendications précédentes, ou un énantiomère, diastéréoisomère, N-oxyde ou sel pharmaceutiquement acceptable d'un tel composé, mélangé avec un diluant ou véhicule pharmaceutiquement acceptable.

7. Procédé pour la préparation d'un composé ayant la formule générale I telle que définie dans la revendication 1, le procédé comprenant l'alkylation d'une amine BH, dans laquelle B est tel que défini dans la revendication 1, avec un produit intermédiaire halogénoalkylé de formule générale II 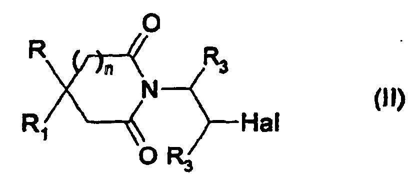 dans laquelle R, R₁, R₃ et n sont tels que définis dans la revendication 1 et Hal représente un atome d'halogène.

8. Procédé selon la revendication 7, dans lequel la réaction est effectuée sans solvant, en présence d'une base, à une température allant de 100 à 180 °C.

9. Procédé pour la préparation d'un composé ayant la formule générale I telle que définie dans la revendication 1, le procédé comprenant l'acylation d'une amine BCH₂CH₂NH₂, dans laquelle B est tel que défini dans la revendication 1, avec un anhydride de formule générale 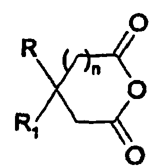 dans laquelle R, R₁ et n sont tels que définis dans la revendication 1.

10. Procédé selon la revendication 9, dans lequel la réaction est effectué dans un solvant aprotique apolaire en présence d'un agent activateur, à une température allant de 80 °C à la température de reflux du solvant.

11. Procédé pour la préparation d'un composé ayant la formule générale I dans laquelle A et A₁ représentent tous les deux des groupes carbonyles, B représente le groupe B₁, R₃ représente un atome d'hydrogène et R, R₁, n et ...... sont tels que définis dans la revendication 1, le procédé comprenant l'amidation d'un diacide de formule générale XXI 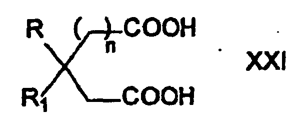 dans laquelle R, R₁ et n sont tels que définis dans la revendication 1, avec une amine BCH₂CH₂NH₂ dans laquelle B est tel que défini dans la revendication 1, et la déshydratation du produit intermédiaire amide obtenu.

12. Procédé selon la revendication 11, dans lequel l'amidation est réalisée dans un solvant aprotique polaire en présence d'un agent de condensation à une température allant de 10 à 40 °C.

13. Procédé selon la revendication 11 et la revendication 12, dans lequel la déshydratation est effectuée en utilisant un anhydride sans solvant, à une température allant de 80 à 120 °C.

14. Procédé pour la préparation d'un composé ayant la formule générale I dans laquelle A représente un groupe méthylène, B représente le groupe B₁ ou B₂, R₃ représente un atome d'hydrogène et A₁, R, R₁, n et ...... sont tels que définis dans la revendication 1, le procédé comprenant la réaction d'un ester aldéhyde de formule générale XXII 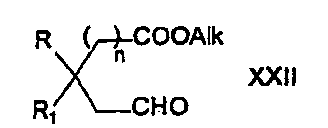 dans laquelle R, R₁ et n sont tels que définis dans la revendication 1, avec une amine BCH₂CH₂NH₂ dans laquelle B est tel que défini dans la revendication 1.

15. Procédé selon la revendication 14, dans lequel la réaction est effectuée dans des conditions réductrices en utilisant un borohydrure de métal, dans un solvant protique polaire, à une température allant de -5 à 25 °C, pour donner un composé de formule I dans laquelle ...... représente une liaison simple et A₁ représente un groupe méthylène.

16. Procédé selon la revendication 14, dans lequel la réaction est effectuée dans un solvant aprotique apolaire en présence d'un catalyseur acide, à la température de reflux du solvant, pour donner un composé de formule I dans laquelle ...... représente une liaison double et A₁ représente un groupe méthyne.

17. Procédé pour la préparation d'un composé ayant la formule générale I telle que définie dans la revendication 1, le procédé comprenant la condensation d'un diester de formule générale 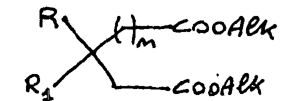 dans laquelle R, R₁ et n sont tels que définis dans la revendication 1, avec une amine BCH₂CH₂NH₂ dans laquelle B est tel que défini dans la revendication 1.

18. Procédé selon la revendication 17, dans lequel la condensation est effectuée sans solvant, à une température allant de 80 à 160 °C.

19. Procédé pour 1a préparation d'un composé ayant la formule générale I dans laquelle A et A₁ représentent tous les deux des groupes méthylènes, ...... représente une liaison simple et B, R, R₁, R₃ et n sont tels que définis dans la revendication 1, le procédé comprenant la réduction d'un composé ayant la formule générale I dans laquelle A et A₁ représentent tous les deux des groupes carbonyles et B, R, R₁, R₃, n et ...... sont tels que définis dans la revendication 1.

20. Procédé selon la revendication 19, dans lequel la réduction est effectuée en utilisant un borohydrure de métal en présence d'un catalyseur de Lewis, dans un solvant aprotique polaire, à une température allant de -5 à 25 °C.
